# EUROPEAN PATENT SPECIFICATION

(11) **EP 1 828 150 B1**
(45) Date of publication and mention of the grant of the patent: **14.03.2012**
(21) Application number: 05854269.7
(22) Date of filing: 15.12.2005
(51) Int. Cl.: C07C 271/58, C07D 239/91, C07D 239/94, C07D 265/24

(54) **PROCESSES FOR PRODUCING 4-AMINOQUINAZOLINES**
VERFAHREN ZUR HERSTELLUNG VON 4-AMINOCHINAZOLINEN
PROCEDES DE PRODUCTION DE 4-AMINOQUINAZOLINES

(30) Priority: 17.12.2004 US 637278 P
(43) Date of publication of application: 05.09.2007
(60) Divisional application: 10181554.6
(73) Proprietor: VERTEX PHARMACEUTICALS INCORPORATED, Cambridge, MA 02139 (US)
(72) Inventor: SILVA, Richard, A., Needham, MA 02492 (US); JONES, Andrew, Needham, MA 02492 (US); BLYTHE, Todd, A., Georgetown, MA 01833 (US)
(74) Representative: Cohausz & Florack
(86) International application number: PCT/US2005/045506
(87) International publication number: WO 2006/066044

(56) References cited:
- WO-A-2004/078733
- PETRIDOU-FISCHER J. ET AL.: JOURNAL OF HETEROCYCLIC CHEMISTRY, vol. 20, 1983, pages 1159-1167, XP002394184

## Description

### TECHNICAL FIELD OF THE INVENTION

The present invention relates to methods of preparing compounds useful as inhibitors of ion channels, and intermediates thereto. WO 2004/078733 A1 describes quinazoline compounds which are useful as inhibitors of voltage-gated sodium channels and calcium channels.

### BACKGROUND OF THE INVENTION

The present invention provides processes for producing 4-amino-quinazolines and analogs thereof. These compounds are useful as inhibitors of voltage-gated sodium channels and calcium channels.

### SUMMARY OF THE INVENTION

As described herein, the present invention provides methods for preparing compounds useful as inhibitors of voltage-gated sodium channels and calcium channels. Such compounds include compounds of formula I: 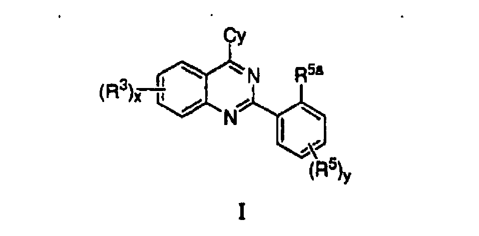 or suitable salts thereof;
wherein Cy, R³, x, R^{5a}, R⁵ and y are as defined in any of the embodiments herein.

The present invention also provides compounds useful as intermediates in the processes of the present invention.

### DETAILED DESCRIPTION OF THE INVENTION

The compounds used in the present invention include compounds of formula **I**: 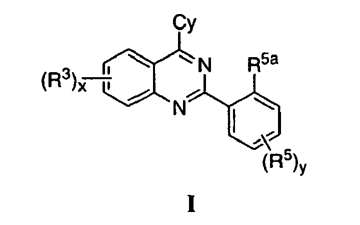 or suitable salts thereof;
wherein:
Cy is a ring selected from: 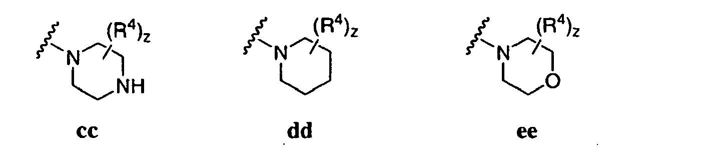 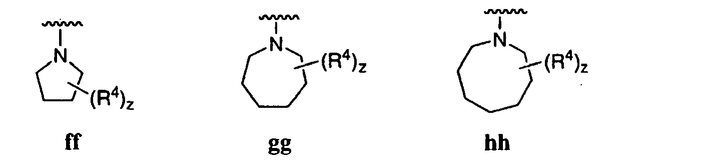 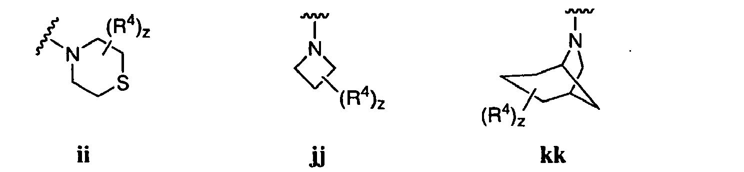 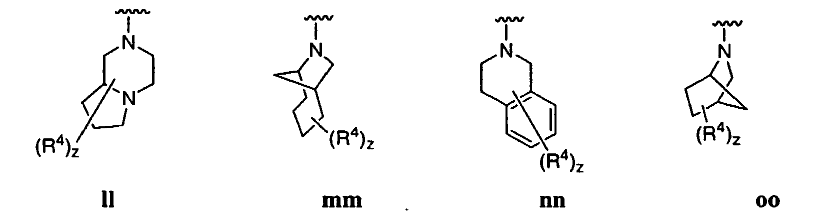 wherein Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl.
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂. -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
R^{5a} is Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂N(CH₃)₂,4-CH₃-piperazin-1-yl, OCOCH(CH₃)₂, OCO(cyclopentyl), -COCH₃, optionally substituted phenoxy, or optionally substituted benzyloxy; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

Compounds used in this invention include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

As described herein, compounds used in the invention may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic C₃-C₈ hydrocarbon or bicyclic C₈-C₁₂ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "heteroaliphatic", as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle", "heterocyclyl", "heterocycloaliphatic", or "heterocyclic" groups.

The term "heterocycle", "heterocyclyl", "heterocycloaliphatic", or "heterocyclic" as used herein means non-aromatic, monocyclic, bicyclic, or tricyclic ring systems in which one or more ring members are an independently selected heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term "unsaturated", as used herein, means that a moiety has one or more units of unsaturation.

The term "alkoxy", or "thioalkyl", as used herein, refers to an alkyl group, as previously defined, attached to the principal carbon chain through an oxygen ("alkoxy") or sulfur ("thioalkyl") atom.

The terms "haloalkyl", "haloalkenyl" and "haloalkoxy" means alkyl, alkenyl or alkoxy, as the case may be, substituted with one or more halogen atoms. The term "halogen" means F, Cl, Br, or I.

The term "aryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring". The term "aryl" also refers to heteroaryl ring systems as defined hereinbelow.

The term "heteroaryl", used alone or as part of a larger moiety as in "heteroaralkyl" or "heteroarylalkoxy", refers to monocyclic, bicyclic, and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic".

An aryl (including aralkyl, aralkoxy, aryloxyalkyl and the like) or heteroaryl (including heteroaralkyl and heteroarylalkoxy and the like) group may contain one or more substituents and thus may be "optionally substituted". Unless otherwise defined above and herein, suitable substituents on the unsaturated carbon atom of an aryl or heteroaryl group are generally selected from halogen; -R°; -OR°; -SR°; phenyl (Ph) optionally substituted with R°; -O(Ph) optionally substituted with R°; -(CH₂)₁₋₂(Ph), optionally substituted with R°; -CH=CH(Ph), optionally substituted with R°; -NO₂; -CN; -N(R°)₂; -NR°C(O)R°; - NR°C(S)R°; -NR°C(O)N(R°)₂; -NR°C(S)N(R°)₂; -NR°CO₂R°; -NR°NR°C(O)R°; -NR°NR°C(O)N(R°)₂; -NR°NR°CO₂R°; -C(O)C(O)R°; -C(O)CH₂C(O)R°; -CO₂R°; - C(O)R°; -C(S)R°; -C(O)N(R°)₂; -C(S)N(R°)₂; -OC(O)N(R°)₂; -OC(O)R°; -C(O)N(OR°) R°; -C(NOR°) R°; -S(O)₂R°; -S(O)₃R°; -SO₂N(R°)₂; -S(O)R°; -NR°SO₂N(R°)₂; -NR°SO₂R°; -N(OR°)R°; -C(=NH)-N(R°)₂; -P(O)₂R°; -PO(R°)₂; -OPO(R)₂; -(CH₂)₀₋₂NHC(O)R°; phenyl (Ph) optionally substituted with R°; -O(Ph) optionally substituted with R°; -(CH₂)₁₋₂(Ph), optionally substituted with R°; or -CH=CH(Ph), optionally substituted with R°; wherein each independent occurrence of R° is selected from hydrogen, optionally substituted C₁₋₆ aliphatic, an unsubstituted 5-6 membered heteroaryl or heterocyclic ring, phenyl, -O(Ph), or -CH₂(Ph), or, notwithstanding the definition above, two independent occurrences of R°, on the same substituent or different substituents, taken together with the atom(s) to which each R° group is bound, to form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group of R° are selected from NH₂, NH(C_{1 4}aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic, wherein each of the foregoing C₁₋₄aliphatic groups of R° is unsubstituted.

An aliphatic or heteroaliphatic group, or a non-aromatic heterocyclic ring may contain one or more substituents and thus may be "optionally substituted". Unless otherwise defined above and herein, suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR*, =NN(R*)₂, =NNHC(O)R*, =NNHCO₂(alkyl), =NNHSO₂(alkyl), or =NR*, where each R* is independently selected from hydrogen or an optionally substituted C₁₋₆ aliphatic group.

Unless otherwise defined above and herein, optional substituents on the nitrogen of a non-aromatic heterocyclic ring are generally selected from -R⁺, -N(R⁺)₂, -C(O)R⁺, -CO₂R⁺, -C(O)C(O)R⁺, -C(O)CH₂C(O)R⁺, -SO₂R⁺, -SO₂N(R⁺)₂, -C(=S)N(R⁺¹)₂, -C(=NH)-N(R⁺)₂, or -NR⁺SO₂R⁺; wherein R⁺ is hydrogen, an optionally substituted C₁₋₆ aliphatic, optionally substituted phenyl, optionally substituted -O(Ph), optionally substituted -CH₂(Ph), optionally substituted -(CH₂)₁₋₂(Ph); optionally substituted -CH=CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of R⁺, on the same substituent or different substituents, taken together with the atom(s) to which each R⁺ group is bound, form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group or the phenyl ring of R⁺ are selected from -NH₂, -NH(C₁₋₄ aliphatic), -N(C₁₋₄ aliphatic)₂, halogen, C₁₋₄ aliphatic, -OH, -O(C₁₋₄ aliphatic), -NO₂, -CN, -CO₂H, -CO₂(C₁₋₄ aliphatic), -O(halo C₁₋₄ aliphatic), or halo(C₁₋₄ aliphatic), wherein each of the foregoing C₁₋₄aliphatic groups of R⁺ is unsubstituted.

The term "alkylidene chain" refers to a straight or branched carbon chain that may be fully saturated or have one or more units of unsaturation and has two points of attachment to the rest of the molecule.

As detailed above, in some embodiments, two independent occurrences of R° (or R⁺, R, R' or any other variable similarly defined herein), are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

Exemplary rings that are formed when two independent occurrences of R° (or R⁺, R, R' or any other variable similarly defined herein), are taken together with the atom(s) to which each variable is bound include, but are not limited to the following: a) two independent occurrences of R° (or R⁺, R, R' or any other variable similarly defined herein) that are bound to the same atom and are taken together with that atom to form a ring, for example, N(R°)₂, where both occurrences of R° are taken together with the nitrogen atom to form a piperidin-1-yl, piperazin-1-yl, or morpholin-4-yl group; and b) two independent occurrences of R° (or R⁺, R, R' or any other variable similarly defined herein) that are bound to different atoms and are taken together with both of those atoms to form a ring, for example where a phenyl group is substituted with two occurrences of OR° 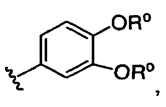 these two occurrences of R° are taken together with the oxygen atoms to which they are bound to form a fused 6-membered oxygen containing ring: 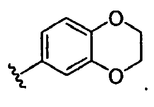 It will be appreciated that a variety of other rings can be formed when two independent occurrences of R° (or R⁺, R, R' or any other variable similarly defined herein) are taken together with the atom(s) to which each variable is bound and that the examples detailed above are not intended to be limiting.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

In certain embodiments, the methods described herein are useful for preparing compounds of formula Ia: 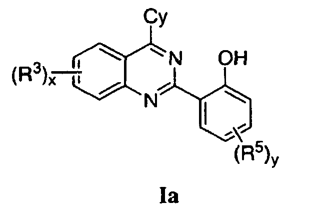 or suitable salts thereof; wherein:
Cy is a ring selected from: 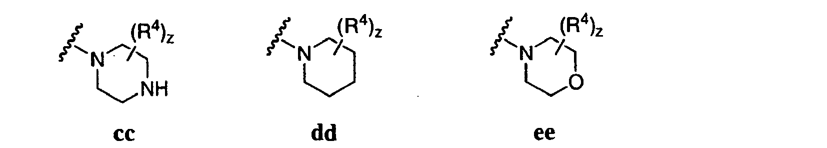 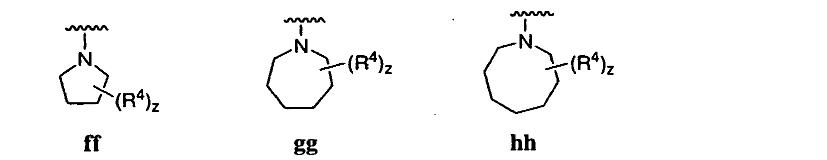 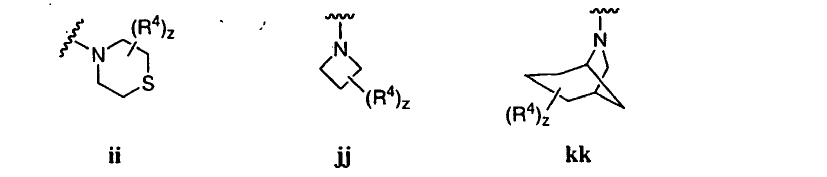 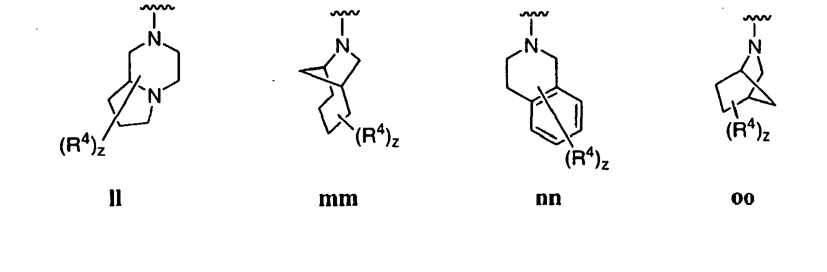 wherein Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')2_{,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl.
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl.
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

In other embodiments, the methods described herein are useful for preparing compounds of formula **Ia:** 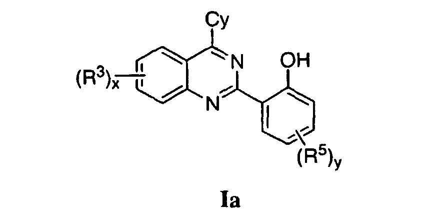 or suitable salts thereof; wherein:
Cy is azetidin-1-yl **(jj),** pyrrolidin-1-yl **(ff),** piperidinl-yl **(dd),** or piperazin-1-yl **(cc),** wherein Cy is optionally substituted with 0-4 occurences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂OCH₃, -CONH₂, -COOCH₃, -OH, -CH₂OH, -NHCOCH₃, -SO₂NH₂, -SO₂(CH₂)₃CH₃, -SO₂CH(CH₃)₂, -SO₂N(CH₃)₂, -SO₂CH₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, -C(O)NHCH(CH₃)₂, -C(O)CH₂CH₃, or an optionally substituted group selected from -piperidinyl, piperizinyl, morpholino; C₁₋₄alkoxy, phenyl, phenyloxy, benzyl, benzyloxy, -CH₂cyclohexyl, pyridyl, -CH₂pyridyl, or -CH₂thiazolyl;
x is 1 or 2;
each occurrence of R³ is independently Cl, Br, F, CF₃, -OCF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, - OCH₂CH₃, -CH₂OH, -NHCOCH₃, -NHCOCH(CH₃)₂, -SO₂NH₂, -CONH(cyclopropyl), - CONHCH₃, -CONHCH₂CH₃, or an optionally substituted group selected from - piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO₂NHC(CH₃)₂, -OCOC(CH₃)₃, -OCOCH₂C(CH₃)₃, - O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

In still other embodiments, the methods described herein are useful for preparing compounds of formula Ia wherein x is 1 and R³ is at the 7-position of the quinazoline ring and is -Cl, -CH₃, -CH₂CH₃, -F, -CF₃, -OCF₃, -CONHCH₃, -CONHCH₂CH₃, - CONH(cyclopropyl), -OCH₃, -NH₂, -OCH₂CH₃, or -CN. In yet other embodiments, x is 1 and R³ is at the 7-position of the quinazoline ring and is -Cl, -CH₃, -CH₂CH₃, -F, -CF₃, - OCF₃, -OCH₃, or -OCH₂CH₃. In certain other embodiments, x is 1 and R³ is at the 7-position of the quinazoline ring and is methyl.

According to another embodiment, Cy is piperazin-1-yl **(cc)**, y is 0, x is 1 and R³ is at the 7-position of the quinazoline ring and is methyl.

According to yet another embodiment, the methods described herein are useful for preparing compounds of formula **Ia:** 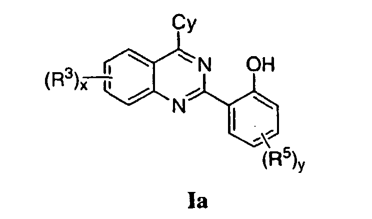 or suitable salts thereof; wherein:
Cy is an optionally substituted ring selected from azetidin-1-yl **(jj),** pyrrolidin-1-yl **(ff),** piperidin1-yl **(dd),** or piperazin-1-yl **(cc),** wherein Cy is optionally substituted with 0-4 occurences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂OCH₃, -CONH₂, -COOCH₃, -OH, -CH₂OH, -NHCOCH₃, -SO₂NH₂, -SO₂(CH₂)₃CH₃, -SO₂CH(CH₃)₂, -SO₂N(CH₃)₂, -SO₂CH₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, -C(O)NHCH(CH₃)₂, -C(O)CH₂CH₃, or an optionally substituted group selected from -piperidinyl, piperizinyl, morpholino, C₁₋₄alkoxy, phenyl, phenyloxy, benzyl, benzyloxy, -CH₂cyclohexyl, pyridyl, -CH₂pyridyl, or -CH₂thiazolyl;
x is 1;
each R³ is independently Cl, Br, F, CF₃, -OCF₃, Me, Et, CN, -COOH, -OH, or -OCH₃; y is 0 or 1; and
each R⁵ is independently Cl, Br, F, CF₃, Me, -OH, -OCH₃, -OCH₂CH₃, -CH₂OH, - NHCOCH₃, -SO₂NH₂, -SO₂NHC(CH₃)₂.

In certain embodiments, the methods described herein are useful for preparing compounds of formula **Ia**: 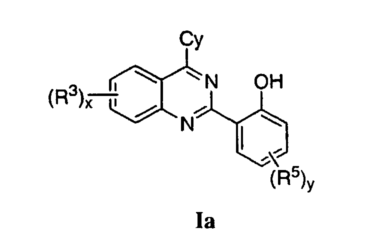 or suitable salts thereof;
wherein:
Cy is unsubstituted piperazin-1-yl, x is 1 and y is 0.

In certain other embodiments, the methods described herein are useful for preparing compounds of formula **Ia:** 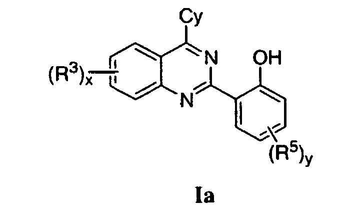 or suitable salts thereof;
wherein:
Cy is piperazin-1-yl optionally substituted on the nitrogen with R⁴, x is 1 and y is 0.

Compounds of formula **Ia** are prepared generally as depicted in Scheme I, below.

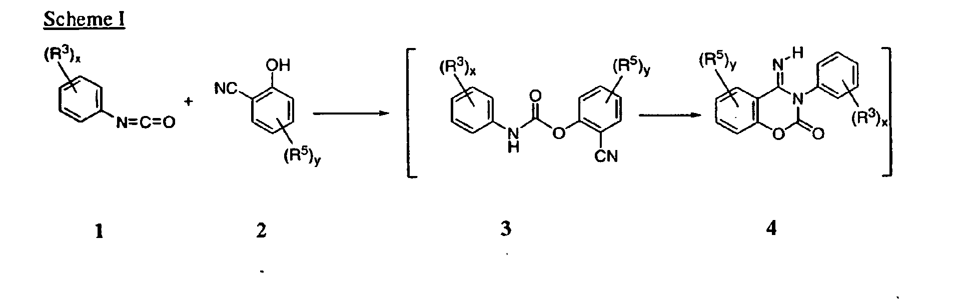

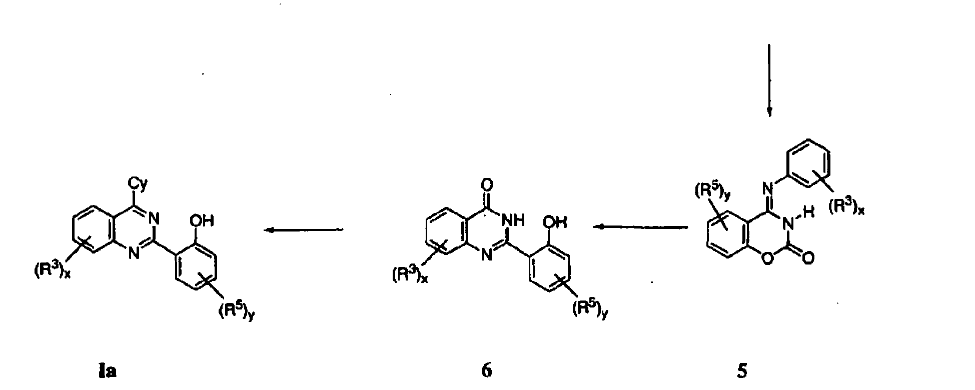

Scheme I above depicts a general method for preparing compounds of formula **Ia.** As is readily apparent, such compounds of formula **Ia** correspond to compounds of formula **I** wherein R^{5a} is -OH. One of ordinary skill in the art would recognize that a variety of compounds of formula **I,** wherein R^{5a} is other than -OH are prepared from intermediate 6 or a suitable salt thereof using methods known in the art. For example, the -OH group of intermediate **6** may be converted to a suitable leaving group. As used herein, a suitable leaving group is a chemical moiety that is readily displaced by a desired incoming chemical moiety. Suitable leaving groups are well known in the art, e.g., see, "Advanced Organic Chemistry," Jerry March, 4th Ed., pp. 351-357, John Wiley and Sons, N.Y. (1992) and "Comprehensive Organic Transformations," Larock, Richard C., 2nd Ed., John Wiley & Sons, 1999.

Such leaving groups include, but are not limited to, halogen, alkoxy, sulphonyloxy, optionally substituted alkylsulphonyl, optionally substituted alkenylsulfonyl, optionally substituted arylsulfonyl, and diazonium moieties.

The suitable leaving group may then be displaced by a variety of moieties to form compounds of formula **I.** Thus, it will be appreciated that after the hydroxyl group of intermediate **6** is converted to a suitable leaving group, a variety of functional groups may be incorporated to form a compound of formula **I** having a variety of R^{5a} groups. For example, said leaving group by be displaced by halogen, a haloalkyl moiety, an alkyl moiety, CN, a carboxylate moiety, NH₃, NH(CH₃)₂, N(Et)₂, NH(iPr)₂, HO(CH₂)₂OCH₃, HCONH₂, HCOOCH₃, HOCH₃, HOCH₂CH₃, HCH₂OH, NH₂COCH₃, HSO₂NH₂, HSO₂NHC(CH₃)₂, HOCOC(CH₃)₃, HOCOCH₂C(CH₃)₃, HO(CH₂)₂N(CH₃)₂, 4-CH₃-piperazin-1-yl, HOCOCH(CH₃)₂, HOCO(cyclopentyl), HCOCH₃, optionally substituted phenoxy, or optionally substituted benzyloxy to form a compound of formula **I**. One of ordinary skill in the art would also recognize that these groups may be activated in order to affect said displacement.

According to another embodiment of the present invention, the methods described herein are useful for preparing compound **Iaa-1** or a suitable salt thereof: 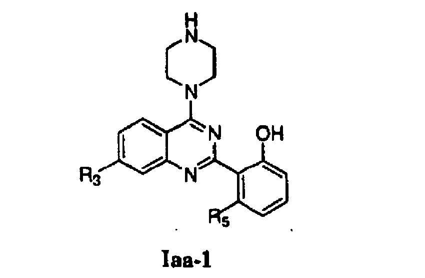 wherein R³ is methyl or hydrogen and R⁵ is fluorine or hydrogen.

According to another embodiment of the present invention, the methods described herein are useful for preparing a compound of formula **V** from a compound of formula **Iaa-1:** 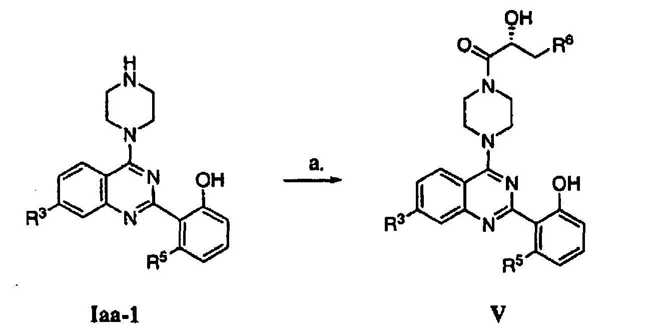 comprising the additional step of:
(a) reacting a compound of formula **Iaa-1** with with a suitable acid auch as (R) isocaproic acid. under suitable amide coupling conditions;
wherein R⁶ is isopropyl or t-butyl, R⁵ is methyl or hydrogen, and R⁵ is fluorine or hydrogen.

In one embodiment of compounds of formula **V,** R⁶ is isopropyl, R³ is methyl, and R⁵ is hydrogen. In another embodiment of formula **V,** R⁶ is t-butyl, R³ is methyl, and R⁵ is hydrogen. In yet another embodiment of formula **V,** R⁶ is isopropyl, R³ is hydrogen, and R⁵ - is hydrogen. In yet another embodiment of formula **V,** R⁶ is t-butyl, R³ is methyl, and R³ is fluorine. Or, in formula **V,** R⁶ is t-butyl, R³ is hydrogen, and R⁵ is fluorine.

In one embodiment, suitable amide coupling conditions include a variety of commonly used organic solvents (such as methylene chloride, THE, ethyl acetate, acetonitrile, DMF, etc.), commercially available amide coupling reagents known to those skilled in the art (such as EDC, BOP, BOP-Cl, DCC, HOBt, etc.), inorganic (such as K₂CO₃, Na₂CO₃, Cs₂CO₃) or organic bases (Et₃N, Hunigs base, N-methylmorpholine, imidazole, 4-DMAP,etc.) and a suitable reaction temperature (from 0°C to greater than 100°C) and a suitable atmosphere (such as air, nitrogen , argon, etc.). In one embodiment for preparing compounds of formula V, the organic solvent in DMF, the the coupling agents are EDC and HOBt, the organic base is 4-methylmorpholine, the atmosphere is nitrogen adn the temperature is room temperature.

In another embodiment, the method further comprises the step of forming a salt of the compound of formula **V.** In one embodiment the salt is a methanesulfonic acid salt.

One of ordinary skill in the art would recognize that compounds of formula **V** may be prepared using methods known in the art. For instance, for preparing compound **V,** wherein R⁶ is isopropyl or t-butyl, the commercially available or synthesized acid. intermediate coupling partner is used along with the suitable amide coupling reagents either with or without added organic or inorganinc base and in a variety of commonly used organic solvents. In one embodiment, wherein R⁶ is isopropyl, one of skill in the art would be able to make the coupling partner isocaproic acid from leucine by known organic chemistry techniques. Finally, one of skill in the art would recognize that the free base of compounds of formula **V** may be converted to a suitable salt for further purification. In one embodiment, the methanesulfonic acid salt is useful for purifying compounds of formula **V.**

In another embodiment, the compound of formula **Ia** is produced as a salt of a sulfonic acid or a dicarboxylic acid. The specific sulfonic acid or dicarboxylic acid useful for producing the salt of compound of formula **Ia** may be selected from acids known in the art. See, e.g., "Practical Process, Research, & Development," Anderson, Neal G., Academic Press, 2000, the contents of which are incorporated herein by reference.

According to one embodiment, the compound of formula **Ia** is produced as a salt of a sulfonic acid. Exemplary sulfonic acids include methylsulfonic acid, p-toluenesulfonic acid, etc. According to one embodiment, the compound of formula **Ia** is produced as a methylsulfonic acid salt. According to another embodiment, the compound of formula **Ia** is produced as a salt of a dicarboxylic acid. In one embodiment, the dicarboxylic acid is selected from oxalic acid, malonic acid, succinic acid, maleic, or fumaric acid. Or, the dicarboxyclic acid is oxalic acid.

In certain embodiments, the present invention provides a method for preparing a compound of formula **Ia:** 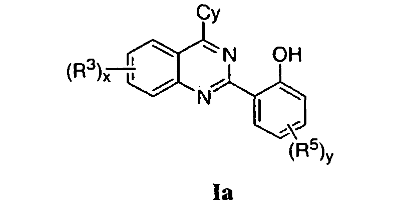 or a suitable salt thereof;
wherein:
Cy is a ring selected from: 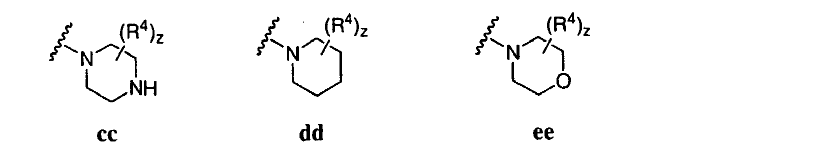 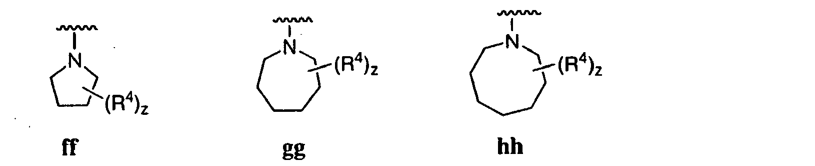 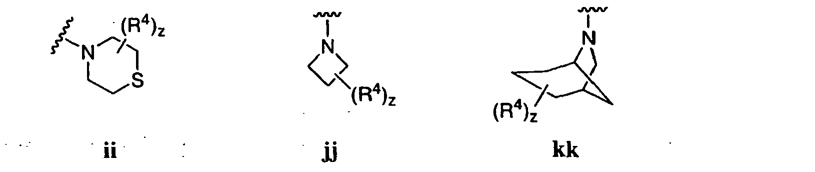 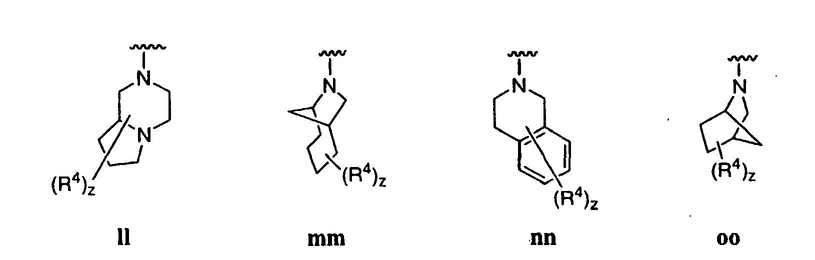 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
   each R⁵ is independently halogen, CN, NO₂, -N(R')₂, -CH₂N(R')₂, -OR', -CH₂OR', - SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR' , -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula **II**: 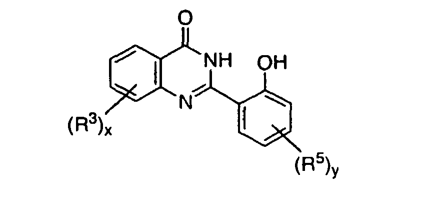 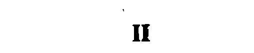 or a suitable salt thereof;
   wherein:
   x is 0-4.;
   each R³ is independently halogen, CN, NO₂, -N(R')₂. -CH₂N(R')₂, -OR', -CH₂OR', -SR',
      - CH₂SR', -COOR', -NRCOR', -CON(R')₂, -OCON(R')₂, COR', -NHCOOR',
      - SO₂R', -SO₂N(R')₂, or an optionally substituted group-selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')2, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
   and
   (b) converting said compound of formula **II** or a suitable salt thereof to a compound of formula **Ia**;
the method of preparing a compound of formula **Ia** or a suitable salt thereof from a compound of formula **II** or a suitable salt thereof further comprises the steps of:
(a) protecting the hydroxyl group of compound **II** with a suitable hydroxyl protecting group to form a compound of formula **IIa:** 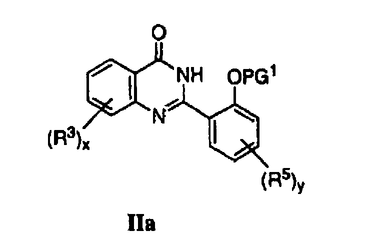 or a suitable salt thereof;
   wherein:
   OPG¹ is an ester group;
   x is 0-4;
   each R³ is independently halogen, CN, NO₂, -N(R')₂. -CH₂N(R')2, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')₂, -CH₂N(R)₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', PO(R')₂, -OPO(R')₂, or an optionally substituted group selected from C₁₋C₆alipllatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(b) converting the the ketone moiety of the compound of formula **IIa** or a suitable salt thereof, to a suitable leaving group to form a compound of formula **IIb**: 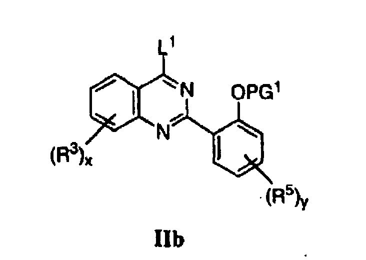 or a suitable salt thereof;
   wherein:
   O PG¹ is an ester group;
   L¹ is a suitable leaving group;
   x is 0-4;
   each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')₂. -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(c) displacing said suitable leaving group with a suitable Cy moiety to form a compound of formula IIc: 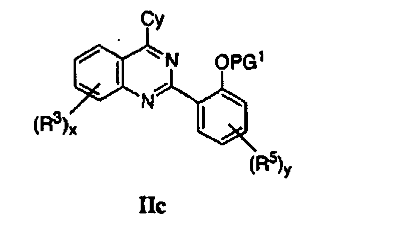 or a suitable salt thereof;
   wherein:
   OPG¹ is an ester group;
   Cy is a ring selected from: 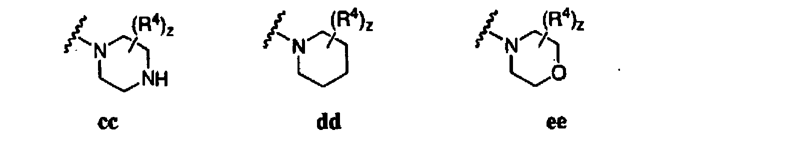 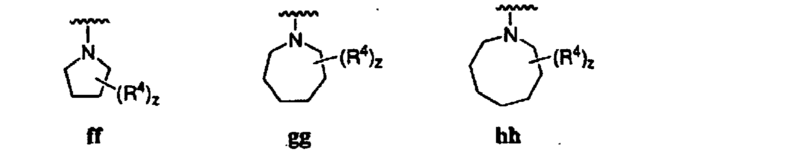 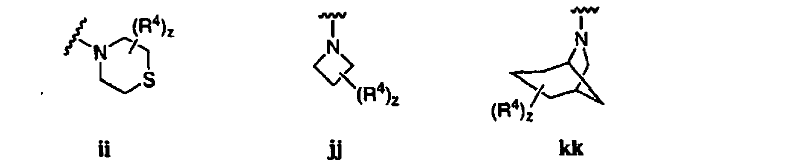 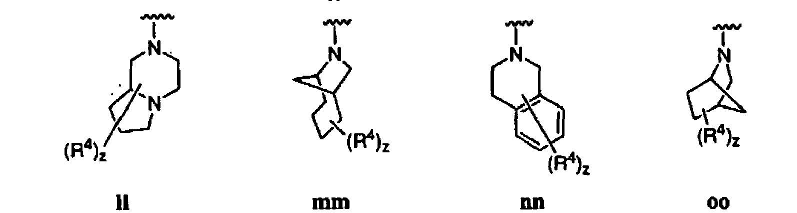 and Cy is optionally substituted at one or more substitutable carbon, nitrogen, or sulfur atoms with z independent occurrences of -R⁴;
   each z is independently 0-5;
   each R⁴ is independently halogen, CN, NO₂, -N(R')₂, -CH₂N(R')₂, -OR', -CH₂OR', -SR', -CH₂SR', -COOR', -NRCOR', -CON(R')2, -OCON(R')₂, COR', -NHCOOR', - SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloatiphaticC₁-C₆alkyl, or heterocycloatiphaticC₁-C₆alkyl;
   x is 0-4;
   each R³ is independently halogen, CN, NO₂, -N(R')₂, -CH₂N(R')₂, -OR', -CH₂OR', -SR', -CH₂SR'. -COOR', -NRCOR', -CON(R')₂. -OCON(R')₂, COR', -NHCOOR', -SO₂R', -SO2N(R')₂, or an optionally substituted group selected from C₁₋C₆aliphatic, aryl, heteroaryl, cycloaliphatic, heterocycloaliphatic, arylC₁-C₄alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
   and
(d) removing the suitable hydroxyl protecting group to form a compound of formula Ia or a suitable salt thereof.

Hydroxyl protecting groups are well known in the art and include those described in detail in "Protecting Groups in Organic Synthesis", T. W. Greene and P. G. M. Wuts, 3rd edition, John Wiley & Sons. 1999. Examples of hydroxyl protecting groups further include, but are not limited to, esters, allyl ethers, ethers, silyl ethers, alkyl ethers, arylalkyl ethers, and alkoxyalkyl ethers. Examples of such esters include formates, acetates, carbonates, and sulfonates. Specific examples include formate, benzoyl formate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (trimethylacetyl), crotonate, 4-methoxy-crotonate, benzoate, p-benylbenzoate, 2,4,6-trimethylbenzoate, carbonates such as methyl, 9-fluorenylmethyl, ethyl, 2,2,2-trichloroethyl, 2-(trimethylsilyl)ethyl, 2-(phenylsulfonyl)ethyl, vinyl, allyl, and p-nitrobenzyl. Examples of such silyl ethers include trimethylsilyl, triethylsilyl, t-butyidimethylsilyl, t-butyldiphenylsilyl, triisopropylsilyl, and other trialkylsilyl ethers. Alkyl ethers include methyl, benzyl, p-methoxybenzyl, 3,4-dimethoxybenzyl, trityl, t-butyl, allyl, and allyloxycarbonyl ethers or derivatives. Alkoxyalkyl ethers include acetals such as methoxymethyl, methylthiomethyl, (2-methoxyethoxy)methyl, benzyloxymethyl, beta-(trimethylsilyl)ethoxymethyl, and tetrahydropyranyl ethers. Examples of arylalkyl ethers include benzyl, p-methoxybenzyl (MPM), 3,4-dimethoxybenzyl, O-nitrobenzyl, p-nitrobenzyl, p-halobenzyl, 2,6-dichlorobenzyl, p-cyanobenzyl, 2- and 4-picolyl. The suitable hydroxyl protecting group OPG1 of compounds of formulae **IIa**, **IIb** and **IIc** is an ester group. In other embodiments, the suitable hydroxyl protecting group OPG1 of compounds of formulae **IIa**, **IIb**, and **IIc** is a pivaloate (trimethylacetyl) group.

Methods of adding and removing such hydroxyl protecting groups are well-known in the art and available, for example, in P. J. Kocienski, Protecting Groups, Thieme, 1994, and in T. W. Greene and P. G. M. Wuts, Protective Groups in Organic Synthesis, 3rd edition, John Wiley & Sons, 1999. One of ordinary skill in the art would recognize that the method appropriate to achieve removal of the protecting group of a compound of formula IIc, at step (d), depends upon the actual protecting groups used and includes those described by Greene. For example, when said hydroxyl protecting group of a compound of formula IIc is an ester group, such removal may be achieved by saponification.

As used herein, a suitable leaving group is a chemical moiety that is readily displaced by a desired incoming chemical moiety. Suitable leaving groups are well known in the art, e.g., see, "Advanced Organic Chemistry," Jerry March, 4th Ed., pp. 351-357, John Wiley and Sons, N.Y. (1992) and "Comprehensive Organic Transformations," Larock, Richard C., 2nd Ed., John Wiley & Sons, 1999. Examples of suitable leaving group L¹ of formula **IIb** include, but are not limited to, halogen, alkoxy, sulphonyloxy, optionally substituted alkylsulphonyl, optionally substituted alkenylsulfonyl, optionally substituted arylsulfonyl, and diazonium moieties. Examples of suitable leaving group L¹ of formula **IIb** include chloro, iodo, bromo, fluoro, methanesulfonyl (mesyl), tosyl, triflate, nitro-phenylsulfonyl (nosyl), and bromo-phenylsulfonyl (brosyl). In certain embodiments, the suitable leaving group L¹ of formula **IIb** is a halogen group. In other embodiments, the suitable leaving group L¹ of formula **IIb** is a chloro group.

According to an alternate embodiment, the suitable leaving group may be generated *in situ* within the reaction medium. For example, a leaving group may be generated *in situ* from a precursor of that compound wherein said precursor contains a group readily replaced by said leaving group *in situ.*

In other embodiments, the preparation of a compound of formula **Ia** from a compound of formula **II** further comprises the step of forming a salt of the compound of formula **Ia**. According to one aspect of the present invention, salt is the oxalic acid salt. According to another aspect of the present invention, the compound of formula **Ia** is treated with oxalic acid to form the oxalic acid salt thereof then that salt is freebased and treated with . methanesulfonic acid to form the mesylate salt of a compound of formula **Ia**.

A compound of formula **Ia**: 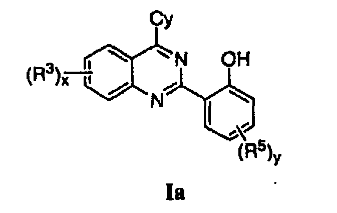 or a suitable salt thereof;
wherein:
Cy is a ring selected from: 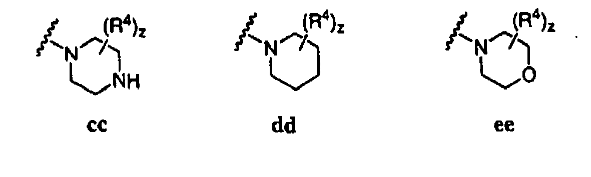 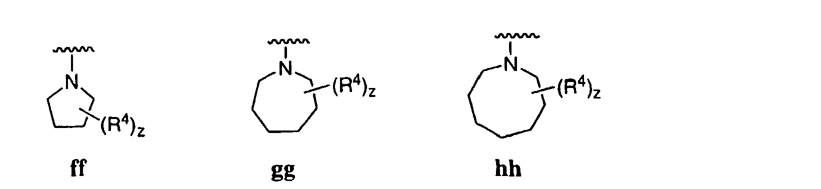 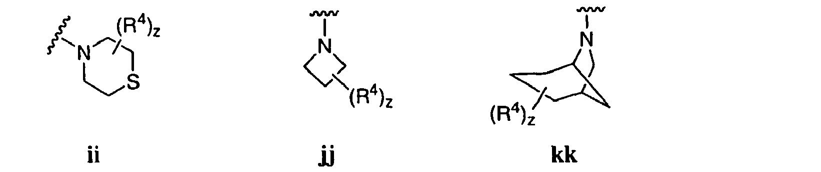 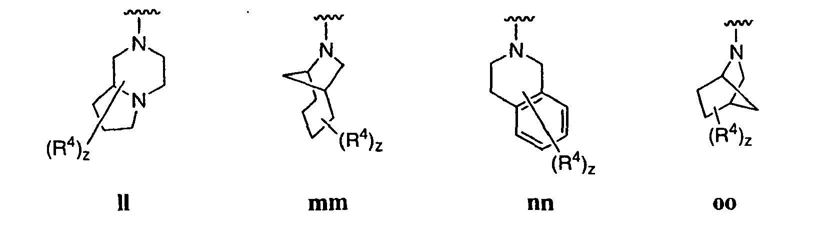 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, NR'SO₂R', -OP(O)(OR')₂, -P(O)(OR')₂, -OP(O)₂OR', -P(O)₂OR', - PO(R')₂, -OPO(R')_{2,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
may be prepared by:
(a) providing a compound of formula **III**: 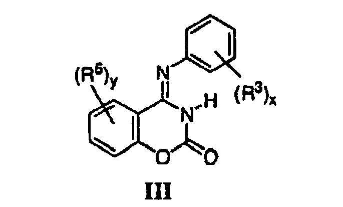 or a suitable salt thereof;
   wherein:
   x is 0-4;
   each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')2, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(b) converting said compound of formula **III** to a compound of formula **II**: 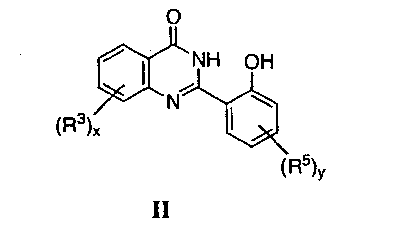 or a suitable salt thereof;
   wherein:
   x is 0-4;
   each R³ is independently halogen, CN, NO₂, -N(R')₂, -CH₂N(R')₂, -OR', -CH₂OR', -SR', -CH₂SR', -COOR', -NRCOR', -CON(R')_{2,} -OCON(R')₂, COR', -NHCOOR', -SO₂R', -SO₂N(R')_{2,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
   y is 0-5;
   each R⁵ is independently halogen, CN, NO₂, -N(R')_{2,} -CH₂N(R')_{2,} -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
      two occurrences of R' are taken together with the atom(s) to which they are bound
         to form an optionally substituted 3-12 membered saturated, partially unsaturated, or fully unsaturated monocyclic or bicyclic ring having 0-4 heteroatoms independently selected from nitrogen, oxygen, or sulfur;
   and
(c) converting said compound of formula **II** to a compound of formula **Ia**.

The conversion of a compound of formula **III** to a compound of formula **II,** at step (b), may be affected by heating. Step (b) may be performed at 150-275 °C. Step (b) may be performed at 200-250 °C in an aprotic solvent. Step (b) may be performed in phenylether.

The present invention uses a compound of formula **II**: 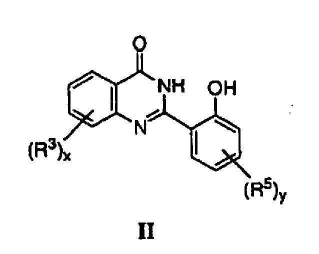 or a suitable salt thereof;
wherein:
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

The present invention uses a compound of formula II: 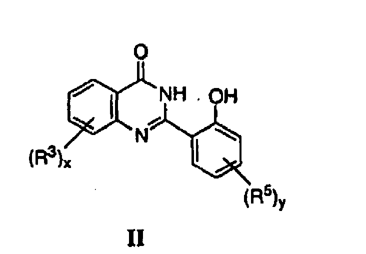 or a suitable salt thereof;
wherein:
x is 0-4;
each R³ is independently halogen, CN, NO₂, -N(R')_{2,} -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')_{2,} -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂,OR', -PO(R')_{2,} -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic
   group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
the following compounds are known from the prior art:
Glycine, N-[2-[2-[6-[bis(carboxymethyl)amino]-2,3-difluorophenoxy]ethoxy]-4-(3,4-dihydro-4-oxo-2-quinazolinyl)-5-hydroxyphenyl]-N-(carboxymethyl)-, tetrapotassium salt;
Glycine, N-[2-[2-[2-[bis(carboxymethyl)amino]-5-(1,4-dihydro-4-oxo-2-quinazolinyl)-4-hydroxyphenoxy]ethoxy]-4-fluorophenyl]-N-(carboxymethyl)-, tetrapotassium salt;
Glycine, N-[2-[2-[2-[bis(2-methoxy-2-oxoethyl)amino]-5-(1,4-dihydro-4-oxo-2-quinazolinyl)-4-hydroxyphenoxy]ethoxy]-4-fluorophenyl]-N-(2-methoxy-2-oxoethyl)-, methyl ester;
Glycine, N-[2-[2-[6-[bis(2-methoxy-2-oxoethyl)amino]-2,3-difluorophenoxy]ethoxy]-4-(3,4-dihydro-4-oxo-2-quinazolinyl)-5-hydroxyphenyl]-N-(2-methoxy-2-oxoethyl)-, methyl ester;
Glycine, N-[2-[2-[2-[bis(carboxymethyl)amino]-5-(1,4-dihydro-4-oxo-2-quinazolinyl)-4-hydroxyphenoxy]ethoxy]-4-methylphenyl]-N-(carboxymethyl)-;
Glycine, N-[2-[2-[2-[bis(carboxymethyl)amino]-5-(1,4-dihydro-4-oxo-2-quinazolinyl)-4-hydroxyphenoxy]ethoxy]-4-fluorophenyl]-N-(carboxymethyl)-;
4(1H)-Quinazolinone, 6-amino-2-(2-hydroxyphenyl)-;
4(1H)-Quinazolinone, 2-(2-hydroxyphenyl)-6-nitro-;
4(1H)-Quinazolinone, 2-(2-hydroxyphenyl)-;
4(1H)-Quinazolinone, 6-chloro-2-(5-chloro-2-hydroxyphenyl)-:
4(1H)-Quinazolinone, 6-butyl-2-(5-butyl-2-hydroxyphenyl)-;
4(1H)-Quinazolinone, 6-bromo-2-(5-bromo-2-hydroxyphenyl)-;
4(1H)-Quinazolinone, 2-(2-hydroxy-5-pentylphenyl)-6-pentyl-;
4(1H)-Quinazolinone, 2-(2-hydroxyphenyl)-;
4(1H)-Quinazolinone, 6-chloro-2-(2-hydroxyphenyl)- ;
4(1H)-Quinazolinone, 2-(2-hydroxyphenyl)-6-methyl-;
4(1H)-Quinazolinone, 6-chloro-2-(5-chloro-2-hydroxyphenyl)-;
4(1H)-Quinazolinone, 2-(2-hydroxyphenyl)-6-iodo-;
4(1H)-Quinazolinone, 2-(5-chloro-2-hydroxyphenyl)-;
4(1H)-Quinazolinone, 2-(2-hydroxy-4-methoxyphenyl)-;
4(1H)-Quinazolinone, 6-chloro-2-(2-hydroxyphenyl)-;
4(1H)-Quinazolinone, 2-(2-hydroxyphenyl)-6-nitro-;
4(1H)-Quinazolinone, 6-chloro-2-(5-chloro-2-hydroxyphenyl)-;
4(1H)-Quinazolinone, 6-chloro-2-(3,5-dichloro-2-hydroxyphenyl)-;
4(1H)-Quinazolinone, 2-(2-hydroxy-5-methoxyphenyl)-;
4(1H)-Quinazolinone, 2-(2-hydroxy-5-nitrophenyl)-;
4(1H)-Quinazolinone, 6-chloro-2-(2-hydroxy-5-methoxyphenyl)-;
4(1H)-Quinazolinone, 2-(2-hydroxy-5-nitrophenyl)-6-nitro-;
4(1H)-Quinazolinone, 6-chloro-2-(2-hydroxy-5-nitrophenyl)-;
4(1H)-Quinazolinone, 2-(3-fluoro-2-hydroxyphenyl)-;
4(1H)-Quinazolinone, 6-chloro-2-(3-fluoro-2-hydroxyphenyl)-;
4(1H)-Quinazotinone, 2-[5-(1,1-dimethylethyl)-2-hydroxyphenyl]-:
4(1H)-Quinazolinone, 2-(4-hydroxy[1,1'-biphenyl]-3-yl)-;
4(1H)-Quinazolinone, 2-(4-chloro-2-hydroxyphenyl)-;
4(1H)-Quinazolinone, 2-(2-hydroxy-3-methylphenyl)-;
4(1H)-Quinazolinone, 2-(3,5-dibromo-2-hydroxyphenyl)-;
4(1H)-Quinazolinone, 8-bromo-2-(3,5-dibromo-2-hydroxyphenyl)-;
4(1H)-Quinazolinone, 6,8-dibromo-2-(3,5-dibromo-2-hydroxyphenyl)-;
4(1H)-Quinazolinone, 2-(3,5-dichloro-2-hydroxyphenyl)-;
4(3H)-Quinazolinone, 2-(4-ethoxy-2-hydroxyphenyl)-;
4(3H)-Quinazolinone, 6-chloro-2-(2-hydroxy-m-tolyl)-;
4(3H)-Quinazolinone, 2-(2-hydroxy-m-tolyl)-6-nitro-;
4(3H)-Quinazolinone, 2-(3,5-dichloro-2-hydroxyphenyl)-6-nitro-;
4(3H)-Quinazolinone, 2-(3,5-dichloro-2-hydroxyphenyl)-6-nitro-;
4(1H)-Quinazolinone, 2-(5-chloro-2-hydroxyphenyl)-6-nitro-;
4(3H)-Quinazolinone, 2-(2-hydroxy-5-iodophenyl)-;
4(3H)-Quinazolinone, 6-chloro-2-(2-hydroxy-5-iodophenyl)-;
4(3H)-Quinazolinone, 2-(2-hydroxy-5-iodophenyl)-6-nitro-;
4(3H)-Quinazolinone, 2-(5-bromo-2-hydroxyphenyly)-6-chloro-;
4(3H)-Quinazolinone, 2-(5-bromo-2-hydroxyphenyl)-6-nitro-;
4(3H)-Quinazolinone, 6-chloro-2-(4-ethoxy-2-hydroxyphenyl)-;
4(3H)-Quinazolinone, 2-(4-ethoxy-2-hydroxyphenyl)-6-nitro-;
4(3H)-Quinazolinone,6-chloro-2-(2,4-dihydroxyphenyl)-;
4(3H)-Quinazolinone, 2-(2,4-dihydroxyphenyl)-6-nitro-;
4(3H)-Quinazolinone, 6-chloro-2-(3,5-dibromo-2-hydroxyphenyl)-;
4(3H)-Quinazolinone, 2-(3,5-dibromo-2-hydroxyphenyl)-6-nitro-;
4(3H)-Quinazolinone, 2-(2-hydroxy-3-biphenylyl)-;
4(3H)-Quinazolinone, 2-(2,5-dihydroxyphenyl)-;
4(3H)-Quinazolinone, 6-chloro-2-(2,5-dihydroxyphenyl)-;
4(3H)-Quinazolinone, 2-(2,5-dihydroxyphenyl)-6-nitro-;
[2-{2-[2-(Carboxymethyl-amino)-5-methyl-phenoxyl-ethoxyl-5-hydroxy-4-(4-hydroxy-quinazolin-2-yl)-phenylamino]-acetic acid; and
[2-{2-[6-(Carboxymethyl-amino)-2,3-difluoro-phenoxy]-ethoxy}-5-hydroxy-4-(4-hydroxy-quinazolin-2-yl)-phenylamino]-acetic acid.

The present invention uses a compound of formula **II**: 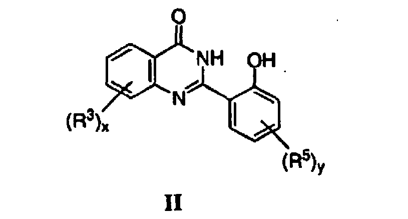 or a suitable salt thereof;
wherein:
x is 1 or 2;
each R³ is independently Cl, Br, F, CF₃, -OCF₃, Me, Et, CN, -COOH, -NH₂, -N(CH₃)₂,-N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH,-NHCOCH₃, -SO₂NH₂, -SO₂NHC(CH₃)₂, -OCOC(CH₃)₃, -OCOCH₂C(CH₃)₃, - O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

The present invention uses a compound of formula **II**: 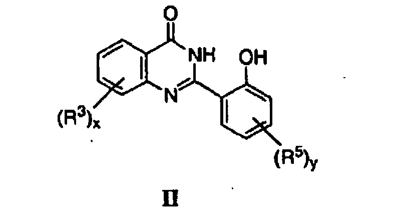 or a suitable salt thereof;
wherein:
x is 1;
each R³ is independently Cl, Br, F, CF₃, -OCF₃, Me, Et, CN, -COOH, -OH, or -OCH₃;
y is 0 or 1; and
each R⁵ is independently Cl, Br, F, CF₃, Me, -OH, -OCH₃, -OCH₂CH₃, -CH₂OH, - NHCOCH₃, -SO₂NH₂, -SO₂NHC(CH₃)₂.

The present invention uses compound **II**-1 or a suitable salt thereof:

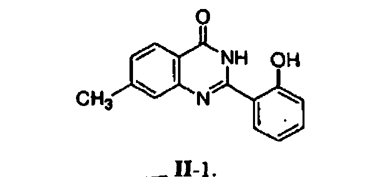

The present invention uses a compound of formula **IIa**: 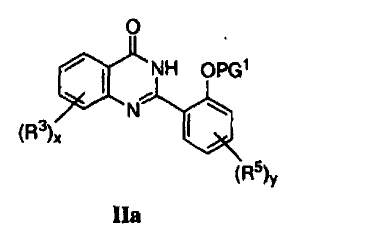 or a suitable salt thereof;
wherein:
OPG¹ is an ester group;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloahphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂. -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloatiphatic, heterocycloaliphatic, arylC₁-C₆alkyl, heteroarylC₁-C₆alkyl, cycloatiphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to
      form an optionally substituted 3-12 membered saturated, partially unsaturated, or fully unsaturated monocyclic or bicyclic ring having 0-4 heteroatoms independently selected from nitrogen, oxygen, or sulfur.

The present invention uses a compound of formula **IIa**: 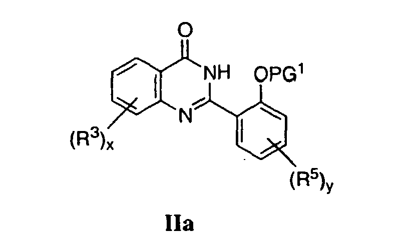 or a suitable salt thereof; wherein:
PG¹ is a suitable hydroxyl protecting group;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')_{2,} -OCON(R')₂, COR', -NHCOOR', -SO₂R', -SO₂N(R')_{2,} or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')_{2,} -OR', -CH₂OR', -SR', -CH₂SR', -NRCOR', -CON(R')_{2,} -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
the following compounds are known from the prior art:
4(1H)-Quinazolinone, 6-chloro-2-[5-chloro-2-(2,2-dimethoxyethoxy)phenyl]-;
4(1H)-Quinazolinone, 2-(2-methoxyphenyl)-;
4(1H)-Quinazolinone, 2-(2,4-dimethoxyphenyl)-;
4(1H)-Quinazolinone, 2-(2,3-dimethoxyphenyl)-;
4(1H)-Quinazolinone, 2-(2,5-dimethoxyphenyl)-;
Benzoic acid, 4-[(aminoiminomethyl)amino]-, 2-(1,4-dihydro-4-oxo-2-quinazolinyl)phenyl ester, monohydrochloride;
4(1H)-Quinazolinone, 2-[2-(acetyloxy)phenyl]-;
4(1H)-Quinazolinone, 2-(2-methoxyphenyl)-;
4(1H)-Quinazolinone, 2-(2-methoxyphenyl)-7-(trifluoromethyl)-;
4(1H)-Quinazolinone, 2-(2-methoxyphenyl)-7-methyl-;
4(1H)-Quinazolinone, 2-[2-(acetyloxy)-5-chlorophenyl]-6-chloro-;
6-Quinazolinecarboxylic acid, 2-(2,3-dimethoxyphenyl)-1,4-dihydro-4-oxo-;
6-Quinazolinecarboxylic acid, 2-(5-ethoxy-2-methoxyphenyl)-1,4-dihydro-4-;
6-Quinazolinecarboxylic acid, 1,4-dihydro-2-[2-methoxy-5-(2-propenyloxy)phenyl]-4-oxo-;
6-Quinazolinecarboxylic acid, 1,4-dihydro-2-(2-methoxy-3-methylphenyl)-4-oxo-;
6-Quinazolinecarboxylic acid, 1,4-dihydro-2-[2-methoxy-5-(1-methylethoxy)phenyl]-4-oxo-;
6-Quinazolinecarboxylic acid, 1,4-dihydro-2-(2-methoxy-5-propoxyphenyl)-4-oxo-;
6-Quinazolinecarboxylic acid 2-[5-(2-ethoxyethoxy)-2-methoxyphenyl]-1,4-dihydro-4-oxo-;
6-Quinazolinecarboxylic acid, 2-(3-ethoxy-2-methoxyphenyl)-1,4-dihydro-4-oxo-;
6-Quinazolinecarboxylic acid, 1,4-dihydro-2-(2-methoxyphenyl)-4-oxo-;
6-Quinazolinecarboxylic acid, 1,4-dihydro-4-oxo-2-[2-(2-propenyloxy)phenyl]-;
6-Quinazolinecarboxylic acid, 2-[2-(2-ethoxyethoxy)phenyl]-1,4-dihydro-4-oxo-;
6-Quinazolinecarboxylic acid, 2-(2,3-dimethoxyphenyl)-1,4-dihydro-4-oxo-;
6-Quinazolinecarboxylic acid, 1,4-dihydro-2-(2-methoxy-3-methylphenyl)-4-oxo-;
6-Quinazolinecarboxylic acid, 1,4-dihydro-2-(2-methoxy-5-methylphenyl)-4-OXO-;
6-Quinazolinecarboxylic acid, 2-[2-(2-ethoxyethoxy)-3-methoxyphenyl]-1,4-dihydro-4-oxo-;
6-Quinazolinecarboxylic acid, 1,4-dihydro-2-(2-methoxyphenyl)-4-oxo-, methyl ester;
4(1H)-Quinazolinone, 6,7,8-trimethoxy-2-(2,3,4-trimethoxyphenyl)-;
Carbonic acid, ethyl ester, ester with 2-(o-hydroxyphenyl)-4(3H)-Quinazolinone;
4(3H)-Quinazolinone, 6-butyl-2-(o-methoxyphenyl)-;
4(1H)-Quinazolinone, 2-(3,5-dibromo-2-methoxyphenyl)-; and
2-(2'-acetoxyphenyl)-4(3H)-quinazolinone.

The x moiety of formula **IIa** may be 1 or 2, and each R³ may be independently Cl, Br, F, CF₃, -OCF₃, Me, Et, CN, -COOH, -NH₂, -N(CH3)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

The x moiety of formula **IIa** may be 1, and R³ may be Cl, Br, F, CF₃, -OCF₃, Me, Et, CN, -COOH, -OH, or -OCH₃.

the y moiety of formula **IIa** may be 0-4, and each R⁵ may be independently Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

The y moiety of formula **IIa** may be 0 or 1, and R⁵ may be Cl, Br, F, CF₃, Me, -OH, -OCH₃, -OCH₂CH₃, -CH₂OH, NHCOCH₃, -SO₂NH₂, - SO₂HC(CH₃)₂.

The PG¹ group of formula **IIa** may be an ester group.

The present invention provides compound IIa-1 or a suitable salt thereof:

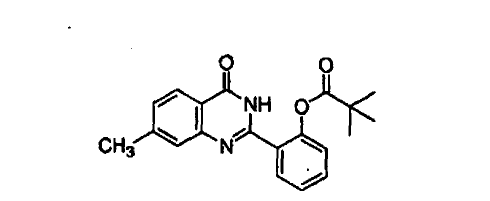

The present invention uses a compound of formula IIb: 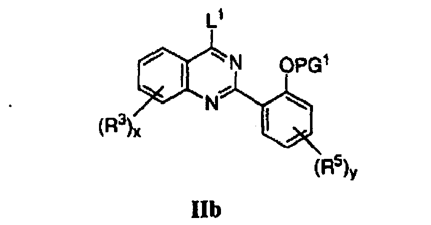 or a suitable salt thereof; wherein:
OPG¹ is an ester group;
L¹ is a suitable leaving group;
x is 0-4;
each R³ is independently halogen, CN, NO₂, -N(R')₂, -CH₂N(R')₂, -OR', -CH₂OR', -SR'. -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR'; -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

The x moiety of formula **IIb** may be 1 or 2, and each R³ may be independently Cl, Br, F, CF₃, -OCF₃, Me, Et, CN, -COOH, -NHz, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

The x moiety of formula **IIb** may be 1, and R³ may be Cl, Br, F, CF₃, -OCF₃, Me, Et, CN, -COOH, -OH, or -OCH₃.

The y moiety of formula I**Ib** may be 0-4, and each R⁵ may independently be Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂(CH₃)₂, 4-CH₃-piperazin-1-yl, OCOCH(CH₃)₂, OCO(cyclopentyl), -COCH₃, optionally substituted phenoxy, or optionally substituted benzyloxy.

The y moiety of formula **IIb** may be 0 or 1, and R⁵ may be Cl, Br, F, CF₃, Me, -OH, -OCH₃, -OCH₂CH₃, -CH₂OH, -NHCOCH₃, -SO₂NH₂, - SO₂NHC(CH₃)₂.

The OPG¹ group of formula **IIb** may be an ester group.

The present invention uses compound **IIb-**1 or a suitable salt thereof:

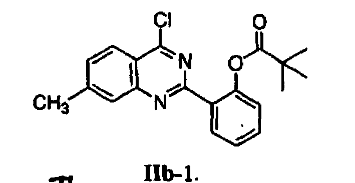

The present invention uses a compound of formula **IIc**: 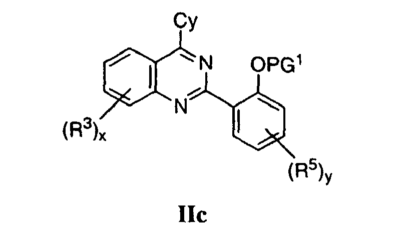 or a suitable salt thereof;
wherein:
PG¹ is a suitable hydroxyl protecting group;
Cy is a ring selected from: 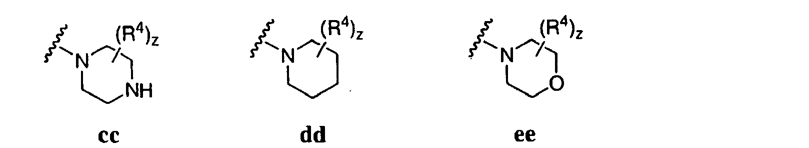 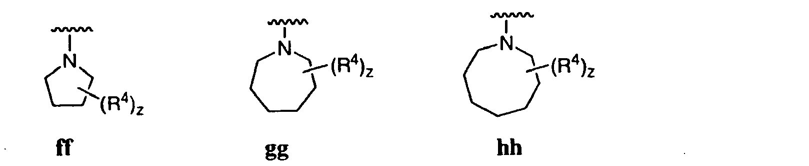 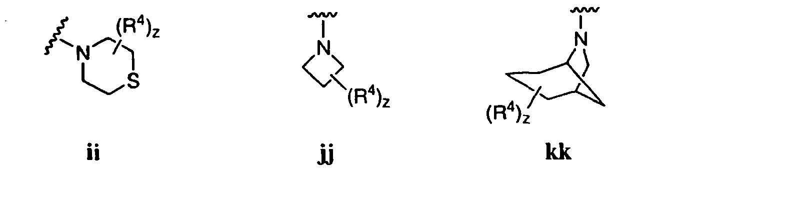 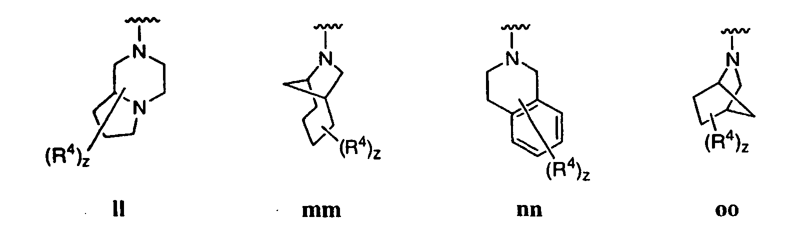
and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁₋₄alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

The present invention uses a compound of formula IIc: 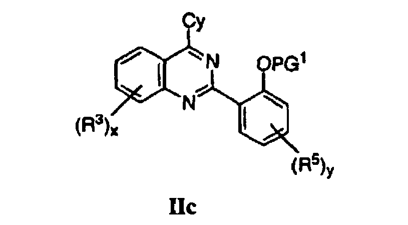 or a suitable salt thereof;
wherein:
OPG¹ is an ester group;
Cy is a ring selected from: 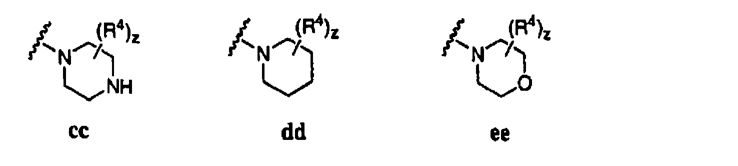 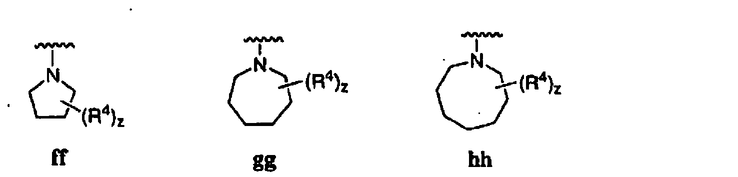 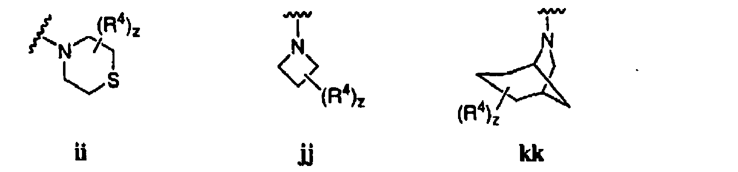 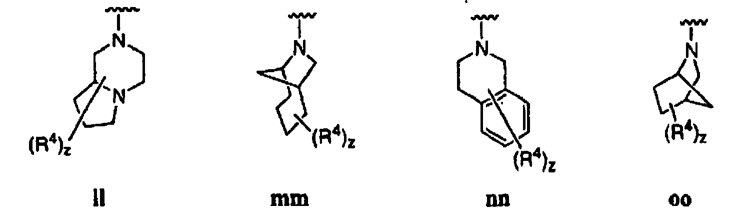
and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR, -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
the following compounds are known from the prior art:
Quinazoline, 2-(2-methoxyphenyl)-4-(1-pyrrolidinyl)-;
Quinazoline, 6-bromo-2-(2-methoxyphenyl)-4-(4-morpholinyl)-;
Quinazoline, 6,8-dichloro-2-(2-methoxyphenyl)-4-(4-morpholinyl)-;
Quinazoline, 6-bromo-2-(2-methoxyphenyl)-4-(1-pyrrolidinyl)-;
Quinazoline, 6,8-dichloro-2-(2-methoxyphenyl)-4-(1-pyrrolidinyl)-;
Quinazoline, 2-(2-fluoro-6-methoxyphenyl)-6-methoxy-4-(4-morpholinyl)-;
Quinazoline, 2-(2-fluoro-6-methoxyphenyl)-4-(4-methyl-1-piperidinyl)-7-(trifluoromethyl)-;
Cyclopropanecarboxylic acid, 3-fluoro-2-[7-methyl-4-(4-methyl-1-piperidinyl)-2-quinazolinyl]phenyl ester;
Propanoic acid, 2-methyl-, 3-fluoro-2-[7-methyl-4-(4-methyl-1-piperidinyl)-2-quinazolinyl]phenyl ester;
Butanoic acid, 3-methyl-, 3-fluoro-2-[7-methyl-4-(4-methyl-1-piperidinyl)-2-quinazolinyl]phenyl ester;
Cyclopentanecarboxylic acid, 3-fluoro-2-[7-methyl-4-(4-methyl-1-piperidinyl)-2-quinazolinyl]phenyl ester;
Propanoic acid, 2,2-dimethyl-, 3-fluoro-2-[7-methyl-4-(4-methyl-1-piperidinyl)-2-quinazolinyl]pbenyl ester;
Butanoic acid, 3,3-dimethyl-, 3-fluoro-2-[7-methyl-4-(4-methyl-1-piperidinyl)-2-quinazolinyl]phenyl ester;
Quinazoline, 7-chloro-2-(2-methoxyphenyl)-4-[3-(trifluoromethyl)-1-pyrrolidinyl];
Piperazine, 1-(butylsulfonyl)-4-[2-(2,4-dimethoxyphenyl)-7-methyl-4-quinazolinyl]-;
Phenol, 3-fluoro-2-[7-methyl-4-(4-memyl-1-piperidinyl)-2-quinazolinyl]-, acetate (ester);
Piperazine, 1-(butylsulfonyl)-4-[2-(2-fluoro-6-methoxyphenyl)-7-(thrifluoromethyl)-4-quinazolinyl]-;
1-Piperazinecarboxylic acid, 4-[6-bromo-2-(2-methoxyphenyl)-4-quinazolinyl]-, 1,1-dimethylethyl ester;
Carbamic acid, (2-methylpropyl)-, 1-[2-(2-methoxyphenyl)-7-methyl-4-quinazolinyl]-4-piperidinyl ester;
6-Quinazolinecarboxylic acid, 4-[4-[(1,1-dimethylethoxy)carbonyl]-1-piperazinyl]-2-(2-methoxyphenyl)-; and
Benzenesulfonamide, 2-methoxy-5-[2-[4-[2-(2-methoxyphenyl)-4-quinazolinyl]-1-piperazinyl]ethyl]-, (2Z)-2-butenedioate (2:3).

The present invention uses a compound of formula **IIc:** 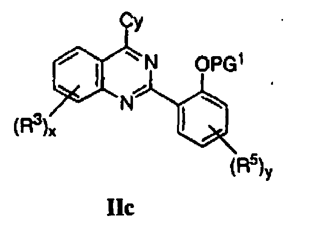 or a suitable salt thereof;
wherein:
OPG¹ is an ester group;
Cy is azetidin-1-yl (**jj**), pyrrolidin-1-yl (**ff**), piperidin1-yl (**dd**), or piperazin-1-yl (**cc**) optionally substituted with 0-4 occurrences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂OCH₃, -CONH₂, -COOCH₃, -OH, -CH₂OH, -NHCOCH₃, -SO₂NH₂, -SO₂(CH₂)₃CH₃, -SO₂CH(CH₃)₂, -SO₂N(CH₃)₂, -SO₂CH₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, -C(O)NHCH(CH₃)₂, -C(O)CH₂CH₃, or an optionally substituted group selected from -piperidinyl, piperizinyl, morpholino, C₁₋₄alkoxy, phenyl, phenyloxy, benzyl, benzyloxy, -CH₂cyclohexyl, pyridyl, -CH₂pyridyl, or -CH₂thiazolyl;
x is 1 or 2;
each R³ is independently Cl, Br, F, CF₃, -OCF₃, Me, Et, CN, -COOH, -NH₂, -N(CH₃)₂, - N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO₂NHC(CH₃)₂, -OCOC(CH₃)₃, -OCOCH₂C(CH₃)₃, - O(CH₂)₂N(CH₃)₂, 4-CH₃-piperazin-1-yl, OCOCH(CH₃)₂, OCO(cyclopentyl), -COCH₃, optionally substituted phenoxy or optionally substituted benzyloxy.

The x moiety of formula **IIc** may be 1 and R³ may be at the 7-position of the quinazoline ring and is -Cl, -CH₃, -CH₂CH₃, -F, -CF₃, -OCF₃, -CONHCH₃, - CONHCH₂CH₃ -CONH(cyclopropyl), -OCH₃, -NH₂, -OCH₂CH₃, or -CN. The x moiety of formula **IIc** may be 1 and R³ may be at the 7-position of the quinazoline ring and is -Cl, -CH₃, -CH₂CH₃, -F, -CF₃, -OCF₃, -OCH₃, or -OCH₂CH₃. The x moiety of formula **IIc** may be 1 and R³ may be at the 7-position of the quinazoline ring and may be methyl.

The present invention uses compound **IIc-1** or a suitable salt thereof:

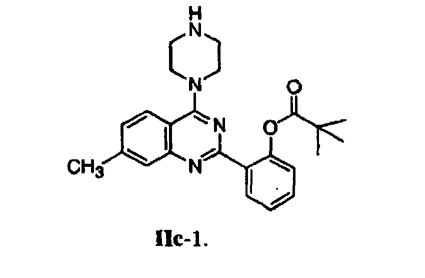

In order that the invention described herein may be more fully understood, the following examples are set forth.

### EXAMPLES

### Example 1

**4-m-Tolylimino-3,4-dihydro-benzo[e][1,3]oxazin-one (III-1):** 2-Cyanophenol (2.4 g) and m-tolylisocyanate (2.6 g) were combined with toluene (10 mL) and triethylamine (3 drops) and the resulting mixture heated at reflux. After 18 hours, the resulting solid was collected to afford 4.0 g of the title compound.

### Example 2

**2-(2-Hydroxy-phenyl)-7-methyl-3H-quinazolin-4-one (II-1):** 4-m-Tolylimino-3,4-dihydro-benzo[e][1,3]oxazin-2-one (1.014 g) was combined with diphenyl ether. The resulting mixture was heated at 250°C for 1 hour. The reaction was allowed to cool and the resulting solid was collected to afford 0.91 g of the title compound as a tan solid. ¹H-NMR (CDCl₃): 8.25 (1H, d), 8.0 (1H, d), 7.55 (1H, s), 7.45 (1H, t), 7.4 (1H, d), 6.85 (1H, d), 6.8 (1H, t).

### Example 3

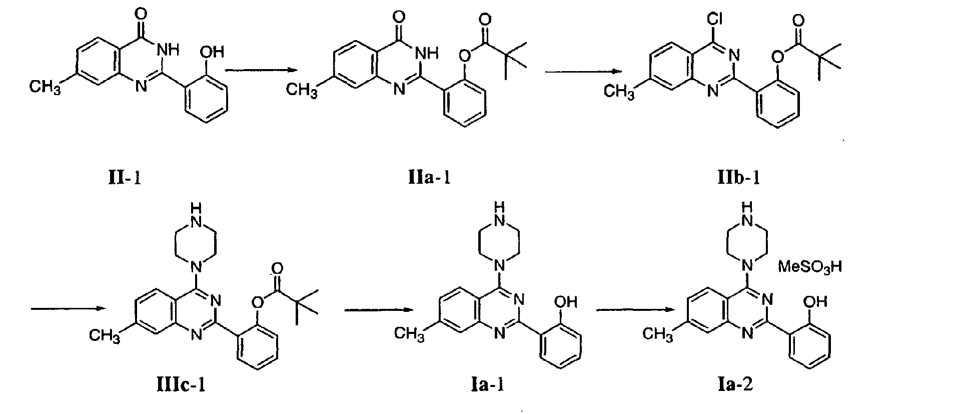

**2,2-Dimethyl-propionic acid 2-(7-methyl-4-oxo-3,4-dihydro-quinazolin-2-yl)-phenyl ester (IIa-1):** 2-(2-Hydroxy-phenyl)-7-methyl-3H-quinazolin-4-one (1.0 g) was suspended in DMF (10 mL). Trimethylacetic anhydride (1.0 mL) and pyridine (0.63 mL) were added and the resulting mixture heated at 50°C for 1 hour. The reaction was poured into water (100 mL) and extracted with methylene chloride (3 x 50 mL). The organic extracts were combined and concentrated *in vacuo* to afford the title compound which was used directly in the next step.

**2,2-Dimethyl-propionic acid 2-(4-chloro-7-methyl-quinazolin-2-yl)-phenyl ester (IIb-1)**: The 2,2-dimethyl-propionic acid 2-(7-methyl-4-oxo-3,4-dihydro-quinazolin-2-yl)-phenyl ester prepared above was dissolved in toluene (10 mL) and treated with phosphoryl oxychloride (0.37 mL) and pyridine (0.63 mL). The resulting solution was stirred at 80°C. The reaction was then poured into ice water and extracted with methylene chloride (3 x 50 mL). The combined organic extracts were concentrated *in vacuo* to approximately 20 mL and this concentrate was used directly in the next step.

**2,2-Dimethyl-propionic acid 2-(7-methyl-4-piperazin-1-yl-quinazolin-2-yl)-phenyl ester (IIIc-1)**: To the concentrate from above was added piperazine (1.36 g) and triethylamine (3.36 mL). The resulting mixture was allowed to stir. The reaction mixture was washed with water then concentrated *in vacuo* and the resulting solid used directly in the next step.

**2-(7-Methyl-4-piperazin-1-yl-quinazolin-2-yl)-phenol (Ia-2) and mesylate salt (Ia-3)**: The solid formed above was dissolved in ethanol (28 mL) and treated with KOH (3.0 g). Upon complete reaction, the mixture was poured into water (100 mL), neutralized with HCl and extracted with methylene chloride (3 x 50 mL). To the resulting solution was added oxalic acid. The resulting solids were collected then freebased and treated with methanesulfonic acid. The resulting solids were recrystallized from ethanol (10 mL) to afford 0.71 g of the mesylate salt as a yellow solid.

### Example 4

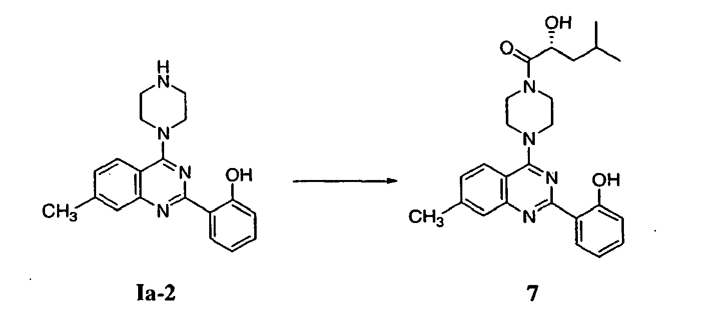

**(R)-2-hydroxy-1-(4-(2-(2-hydroxyphenyl)-7-methylquinazolin-4-yl)piperazin-. 1-yl)-4-methylpentan-1-one (7)**: A mechanically stirred suspension of 2-(7-methyl-4-piperazin-1-yl-quinazolin-2-yl)-phenol (**Ia-2**) in DMF (3.6 liters) and N-methylmorpholine (214 ml, 1.2 equivalents) was treated with 1-(3-dimethylaminopropyl)-3-ethylcarbodiimide HCl (EDC, 200g, 1.2 equivalents) and 1-hydroxybenzotriazole hydrate (HOBt, 160g, 1.2 equivalents). A solution of (R)-isocaproic acid (140g, 1.2 equivalents, prepared by methods know in the art from D-leucine) in dimethylformamide (400 ml) was added to the mixture over a period of 6 hours. The mixture was cooled to 0-5°C and water (8 liters) was added slowly to the mixture. The resulting solid was removed from the reaction mixture both manually and via filtration and the solid was then dissolved in THF (2.0 liters), filtered, and treated with methane sulfonic acid (1.2 equivalents). The resulting solid salt was filtered, then dissolved with heating in anhydrous ethanol (3A grade, 4 liters), cooled to 45°C, held at that temperature for 1 hour and then allowed to cool to room temperature. The resulting mesylate was collected by filtration. The salt was then suspended in methylene chloride (2 liters) and treated with potassium carbonate (145.12g, 2 equivalents) previously dissolved in water (2 liters). The organic layer was collected and evaporated to afford a yellow solid, which after drying under vacuum (125 Torr, room temperature) gave 261.2g of desired product 5 (63% yield) as a yellow solid and as the free base with consistent analytical data.

## Claims

1. A method for preparing a compound of formula **Ia:** 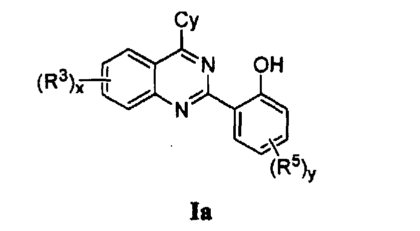 or a suitable salt thereof;
wherein:
Cy is a ring selected from: 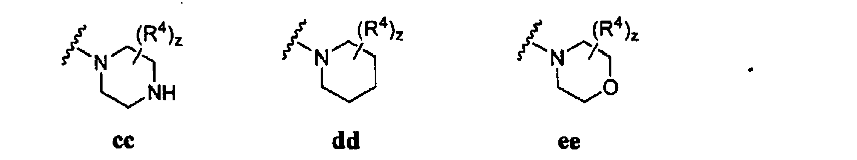 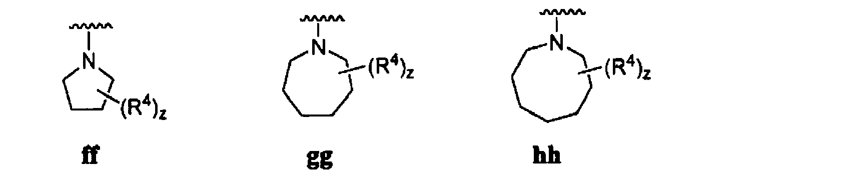 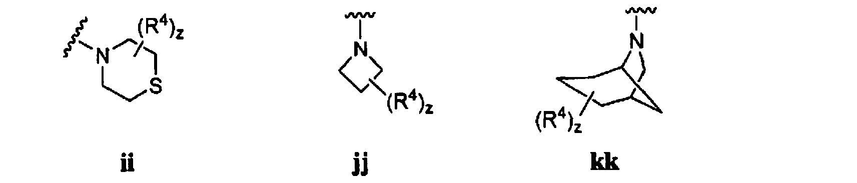 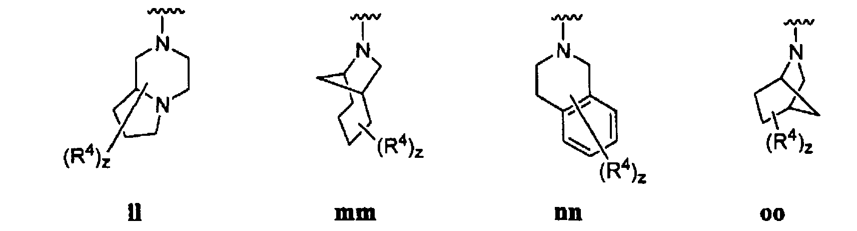 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', - SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula **II**: 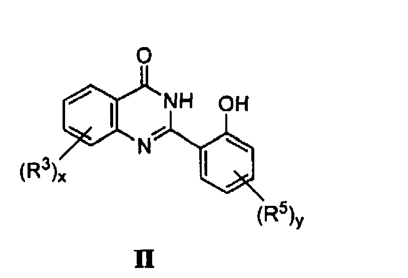 or a suitable salt thereof;
wherein:
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R)₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloatiphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, .S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycioaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
and
(b) converting said compound of formula **II** or a suitable salt thereof, to a compound of formula **Ia** or a suitable salt thereof;
the method further comprising the steps of:
(a) protecting the hydroxyl group of compound **II** with a suitable hydroxyl protecting group to form a compound of formula **IIa:** 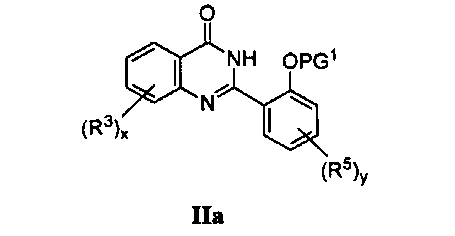 or a suitable salt thereof;
wherein:
OPG¹ is an ester group;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(b) converting the ketone moiety of the compound of formula **IIa** or a suitable salt thereof, to a suitable leaving group to form a compound of formula **IIb**: 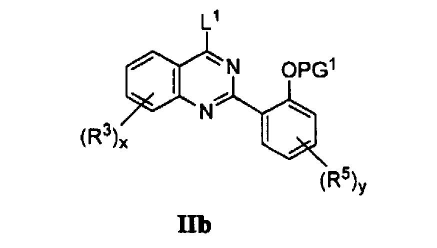 or a suitable salt thereof;
wherein:
OPG¹ is an ester group;
L¹ is a suitable leaving group;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphadc, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(c) displacing said suitable leaving group with a suitable Cy moiety to form a compound of formula **IIc**: 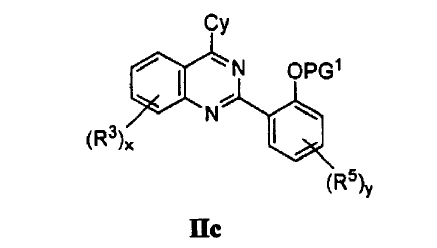 or a suitable salt thereof; wherein:
OPG¹ is an ester group;
Cy is a ring selected from: 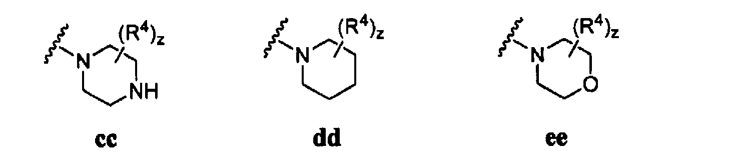 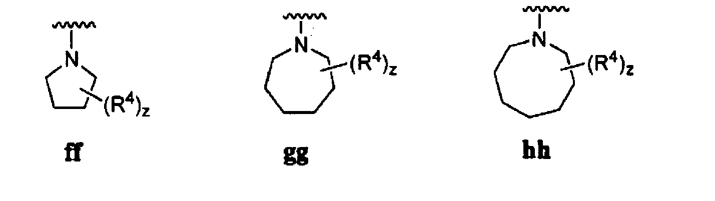 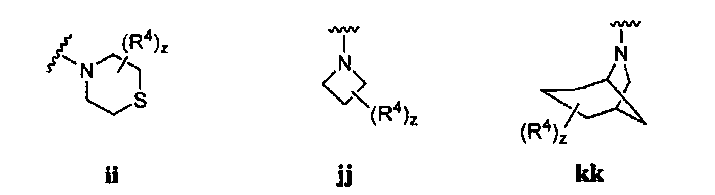 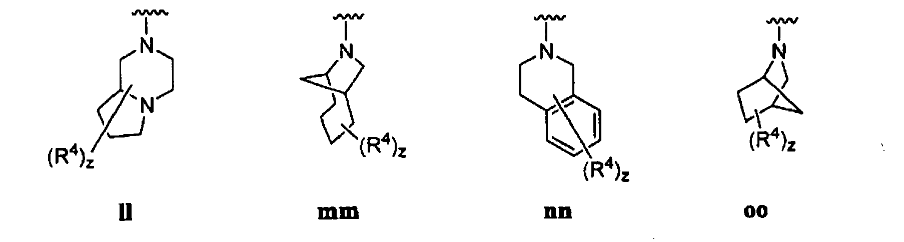 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphatieC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR', -P(O)₂OR', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
and
(d) removing the suitable hydroxyl protecting group to form a compound of formula **Ia**.

2. The method according to claim 1, wherein said ester group is trimethylacetyloxy.

3. The method according to claim 1 or 2, wherein said method further comprises the step of forming a salt of the compound of formula **Ia**.

4. The method according to claim 3, wherein the compound of formula **Ia** is produced as a salt of a sulfonic acid.

5. The method according to claim 3, wherein the compound of formula **Ia** is produced as a salt of methylsulfonic acid or p-toluenesulfonic acid.

6. The method according to claim 3, wherein the compound of formula **Ia** is produced as a salt of methylsulfonic acid.

7. The method according to claim 3, wherein the compound of formula **Ia** is produced as a salt of a dicarboxylic acid.

8. The method according to claim 3, wherein the compound of formula **Ia** is produced as a salt of oxalic acid, malonic acid, succinic acid, maleic acid or fumaric acid.

9. The method according to claim 3, wherein the compound of formula **Ia** is produced as a salt of oxalic acid.

10. The method according to claim 1 to prepare a compound of formula **V** from a compound of formula **Iaa-1**: 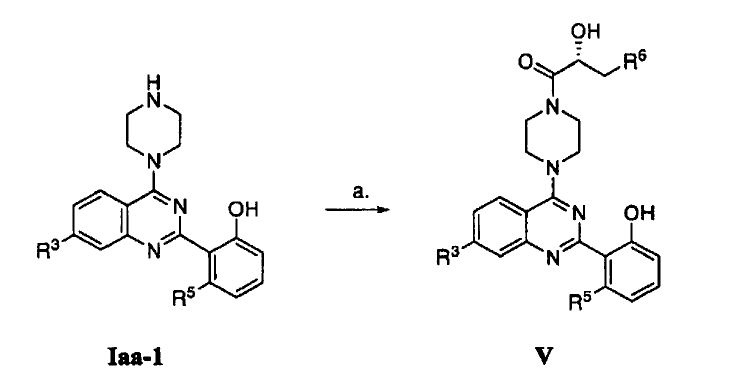 comprising the additional step of:
(a) reacting compound of formula **Iaa-1** with a suitable acid under suitable amide coupling conditions;
wherein R⁶ is isopropyl or t-butyl, R³ is methyl or hydrogen, and R⁵ is fluorine or hydrogen.

11. The method according to claim 10, wherein said method further comprises the step of forming a salt of the compound of formula **V**.

12. The method according to claim 10 or 11, wherein R⁶ is isopropyl, R³ is methyl, and R⁵ is hydrogen.

13. The method according to claim 10 or 11, wherein R⁶ is t-butyl, R³ is methyl, and R⁵ is hydrogen.

14. The method according to claim 10 or 11, wherein R⁶ is isopropyl, R³ is hydrogen, and R⁵ is hydrogen.

15. The method according to claim 10 or 11, wherein R⁶ is t-butyl, R³ is methyl, and R⁵ is fluorine.

## Patentansprüche

1. Verfahren zur Herstellung einer Verbindung mit der Formel **Ia** : 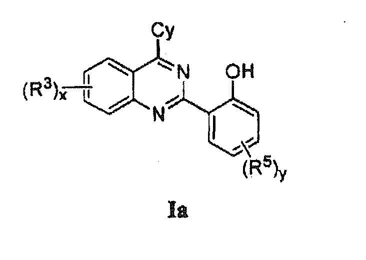 oder eines geeigneten Salzes davon, wobei:
- Cy einen Ring bedeutet, der ausgewählt ist aus: 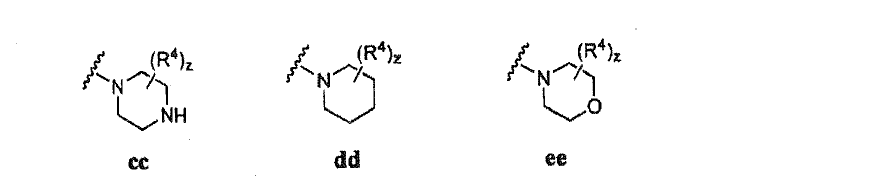 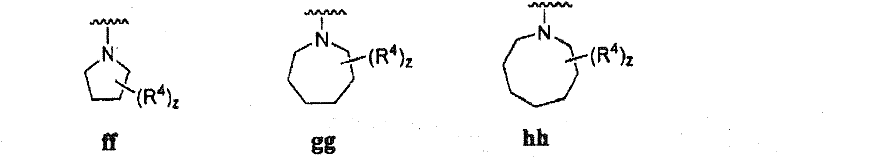 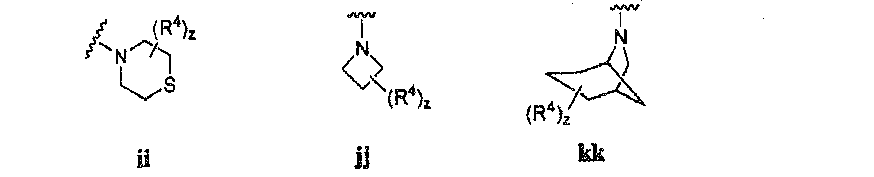 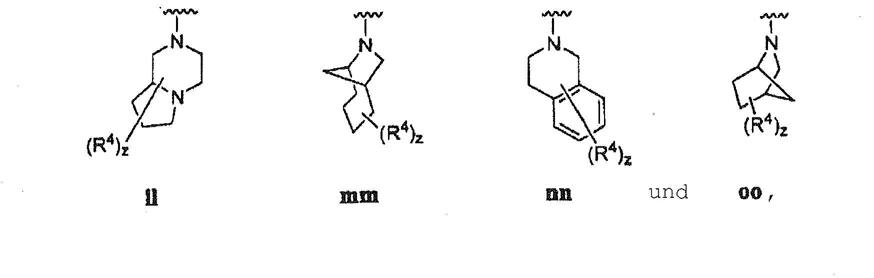 und Cy wahlweise an einem oder mehreren substituierbaren Kohlenstoff-, Stickstoff- oder Schwefelatomen mit z voneinander unabhängigen Vorkommen von -R⁴ substituiert ist, wobei
- jedes z voneinander unabhängig 0 bis 5 und
- jeder R⁴ unabhängig voneinander Halogen, CN, NO₂, -N (R')₂, -CH₂N (R')₂, -OR' , -CH₂OR', -SR' , -CH₂SR', -COOR' , -NRCOR', -CON (R')₂, -OCON (R')₂, COR' , -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist, bedeutet,
- x 0 bis 4,
- jeder R³ unabhängig voneinander Halogen, CN, NO₂, -N (R')₂, -CH₂N (R')₂, -OR' , -CH₂OR', -SR', -CH₂SR', -COOR', -NRCOR', -CON(R')₂, -OCON(R')₂, COR', -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist,
- y 0 bis 5 und
- jeder R⁵ unabhängig voneinander Halogen, CN, NO₂, -N(R')₂, -CH₂N(R')₂, -OR', -CH₂OR', -SR', -CH₂SR', -NRCOR' , -CON(R')₂, -S(O)₂N(R')₂, -OCOR' , -COR' , -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂ oder eine wahlweise substituierte Gruppe, die aus C₁- bis C₆-aliphatisch; Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist, bedeutet, wobei
- jedes Vorkommen von R' unabhängig voneinander Wasserstoff oder eine wahlweise substituierte C₁- bis C₆-aliphatische Gruppe, einen 3- bis 8gliedrigen gesättigten, teilweise ungesättigten oder vollständig ungesättigten monocyclischen Ring mit 0 bis 3 Heteroatomen, die unabhängig voneinander aus Stickstoff, Sauerstoff oder Schwefel ausgewählt sind, oder ein 8- bis 12gliedriges gesättigtes, teilweise ungesättigtes oder vollständig ungesättigtes bicyclisches Ringsystem mit 0 bis 5 Heteroatomen, die unabhängig voneinander aus Stickstoff, Sauerstoff oder Schwefel ausgewählt sind, bedeutet oder
- zwei Vorkommen von R' mit dem/den Atom/en, an welche/s sie gebunden sind, zusammengenommen werden, um einen wahlweise substituierten 3- bis 12gliedrigen gesättigten, teilweise ungesättigten oder vollständig ungesättigten monocyclischen oder bicyclischen Ring mit 0 bis 4 Heteroatomen, die unabhängig voneinander aus Stickstoff, Sauerstoff oder Schwefel ausgewählt sind, zu bilden,
welches die Stufen:
a) Bereitstellen einer Verbindung mit der Formel **II:** 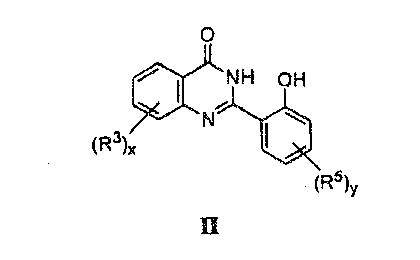 oder eines geeigneten Salzes davon, wobei
- x 0 bis 4,
- jeder R³ unabhängig voneinander Halogen, CN, NO₂, -N (R')₂, -CH₂N (R')₂, -OR' , -CH₂OR', -SR' , -CH₂SR', -COOR', -NRCOR', -CON(R')₂, -OCON(R')₂, COR', -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist,
- y 0 bis 5 und
- jeder R⁵ unabhängig voneinander Halogen, CN, NO₂, -N(R')₂, -CH₂N(R')₂, -OR', -CH₂OR' , -SR' , -CH₂SR' , -NRCOR', -CON(R')₂, -S(O)₂N(R')₂, -OCOR' , -COR', -CO₂R', -OCON (R')₂, -NR'SO₂R', -OP(O)(OR')₂, -P(O)(OR')₂, -OP(O)₂OR', -P(O)₂OR', -PO(R')₂, -OPO(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C₆-Alkyl, HeteroarylC₁- bis C₆-Alkyl, cycloaliphatischC₁-bis C₆-Alkyl oder heterocycloaliphatischC₁- bis C₆-Alkyl ausgewählt ist, bedeutet, wobei
- jedes Vorkommen von R' unabhängig voneinander Wasserstoff oder eine wahlweise substituierte C₁- bis C₆-aliphatische Gruppe, einen 3- bis 8gliedrigen gesättigten, teilweise ungesättigten oder vollständig ungesättigten monocyclischen Ring mit 0 bis 3 Heteroatomen, die unabhängig voneinander aus Stickstoff, Sauerstoff oder Schwefel ausgewählt sind, oder ein 8- bis 12gliedriges gesättigtes, teilweise ungesättigtes oder vollständig ungesättigtes bicyclisches Ringsystem mit 0 bis 5 Heteroatomen, die unabhängig voneinander aus Stickstoff, Sauerstoff oder Schwefel ausgewählt sind, bedeutet oder
zwei Vorkommen von R' mit dem/den Atom/en, an welche/s sie gebunden sind, zusammengenommen werden, um einen wahlweise substituierten 3- bis 12gliedrigen gesättigten, teilweise ungesättigten oder vollständig ungesättigten monocyclischen oder bicyclischen Ring mit 0 bis 4 Heteroatomen, die unabhängig voneinander aus Stickstoff, Sauerstoff oder Schwefel ausgewählt sind, zu bilden,
und
b) Umwandeln der Verbindung mit der Formel **II** oder eines geeigneten Salzes davon in eine Verbindung mit der Formel **Ia** oder ein geeignetes Salz davon umfasst, wobei das Verfahren ferner die Stufen:
a) Schützen der Hydroxylgruppe der Verbindung **II** mit einer geeigneten Hydroxylschutzgruppe, um eine Verbindung mit der Formel **IIa** 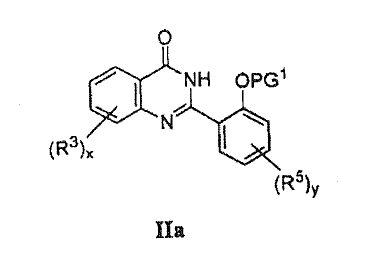 oder ein geeignetes Salz davon zu bilden, wobei:
- OPG¹ eine Estergruppe,
- x 0 bis 4,
- jeder R² unabhängig voneinander Halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', 2-SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist,
- y 0 bis 5 und
- jeder R⁵ unabhängig voneinander Halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR' , -COR' , -CO₂R' , -OCON(R')₂, -NR'SO₂R', -OP(O) (OR')₂, -P(O) (OR')₂, -OP(O)₂OR', -P(O)₂OR', -PO(R')₂, -OPO(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C₆-Alkyl, HeteroarylC₁- bis C₆-Alkyl, cycloaliphatischC₁-bis C₆-Alkyl oder heterocycloaliphatischC₁- bis C₆-Alkyl ausgewählt ist, bedeutet, wobei
- jedes Vorkommen von R' unabhängig voneinander Wasserstoff oder eine wahlweise substituierte C₁- bis C₆-aliphatische Gruppe, einen 3- bis 8gliedrigen gesättigten, teilweise ungesättigten oder vollständig ungesättigten monocyclischen Ring mit 0 bis 3 Heteroatomen, die unabhängig voneinander aus Stickstoff, Sauerstoff oder Schwefel ausgewählt sind, oder ein 8- bis 12gliedriges gesättigtes, teilweise ungesättigtes oder vollständig ungesättigtes bicyclisches Ringsystem mit 0 bis 5 Heteroatomen, die unabhängig voneinander aus Stickstoff, Sauerstoff oder Schwefel ausgewählt sind, bedeutet oder
- zwei Vorkommen von R' mit dem/den Atom/en, an welche/s sie gebunden sind, zusammengenommen werden, um einen wahlweise substituierten 3- bis 12gliedrigen gesättigten, teilweise ungesättigten oder vollständig ungesättigten monocyclischen oder bicyclischen Ring mit 0 bis 4 Heteroatomen, die unabhängig voneinander aus Stickstoff, Sauerstoff oder Schwefel ausgewählt sind, zu bilden,
b) Umwandeln der Keton-Grundeinheit der Verbindung mit der Formel **IIa** oder eines geeigneten Salzes davon in eine geeignete abspaltbare Gruppe, um eine Verbindung mit der Formel **IIb** 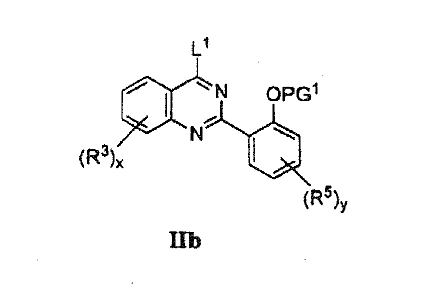 oder ein geeignetes Salz davon zu bilden, wobei:
- OPG¹ eine Estergruppe,
- L¹ eine geeignete abspaltbare Gruppe,
- x 0 bis 4,
- jeder R³ unabhängig voneinander Halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist,
- y 0 bis 5 und
- jeder R⁵ unabhängig voneinander Halogen, CN, NO₂, -N(R')₂, -CH₂N(R')2, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O) (OR')₂, -P(O)(OR')₂, -OP(O)₂OR', -P(O)₂OR', -PO(R')₂, -OPO(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C₆-Alkyl, HeteroarylC₁- bis C₆-Alkyl, cycloaliphatischC₁-bis C₆-Alkyl oder heterocycloaliphatischC₁- bis C₆-Alkyl ausgewählt ist, bedeutet, wobei
- jedes Vorkommen von R' unabhängig voneinander Wasserstoff oder eine wahlweise substituierte C₁- bis C₆-aliphatische Gruppe, einen 3- bis 8gliedrigen gesättigten, teilweise ungesättigten oder vollständig ungesättigten monocyclischen Ring mit 0 bis 3 Heteroatomen, die unabhängig voneinander aus Stickstoff, Sauerstoff oder Schwefel ausgewählt sind, oder ein 8- bis 12gliedriges gesättigtes, teilweise ungesättigtes oder vollständig ungesättigtes bicyclisches Ringsystem mit 0 bis 5 Heteroatomen, die unabhängig voneinander aus Stickstoff, Sauerstoff oder Schwefel ausgewählt sind, bedeutet oder
- zwei Vorkommen von R' mit dem/den Atom/en, an welche/s sie gebunden sind, zusammengenommen werden, um einen wahlweise substituierten 3- bis 12gliedrigen gesättigten, teilweise ungesättigten oder vollständig ungesättigten monocyclischen oder bicyclischen Ring mit 0 bis 4 Heteroatomen, die unabhängig voneinander aus Stickstoff, Sauerstoff oder Schwefel ausgewählt sind, zu bilden,
c) Verdrängen der geeigneten abspaltbaren Gruppe durch eine geeignete Cy-Grundeinheit, um eine Verbindung mit der Formel **IIc** 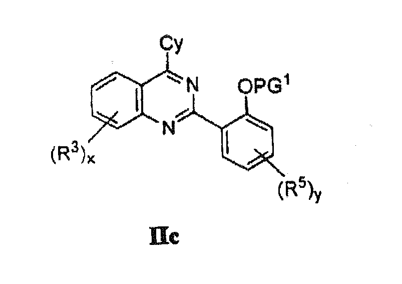 oder ein geeignetes Salz davon zu bilden, wobei:
- OPG¹ eine Estergruppe,
- Cy einen Ring, der ausgewählt ist aus: 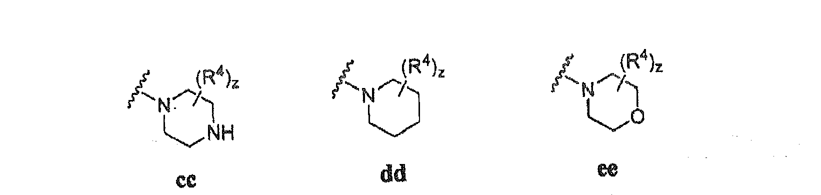 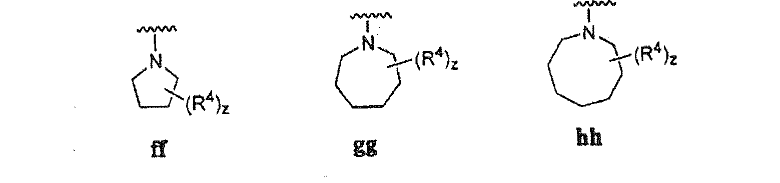 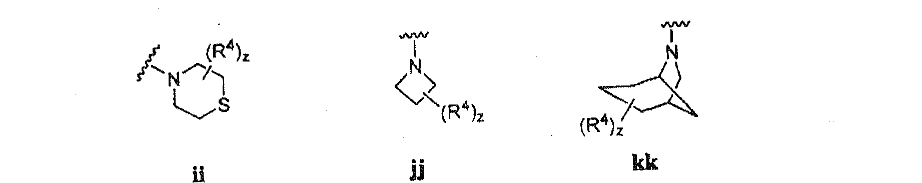 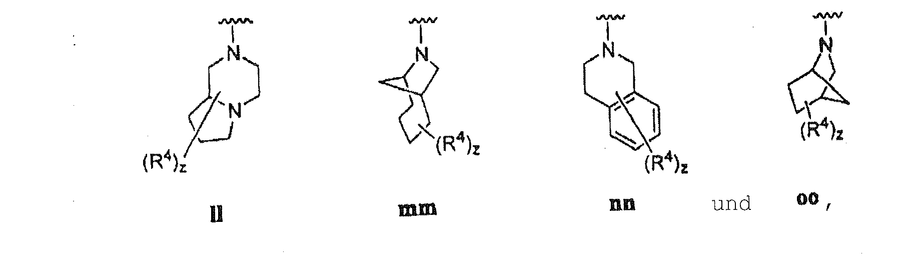 und Cy an einem oder mehreren substituierbaren Kohlenstoff-, Stickstoff- oder Schwefelatomen mit z voneinander unabhängigen Vorkommen von -R⁴ wahlweise substituiert ist, wobei
- z voneinander unabhängig 0 bis 5 und
- jeder R⁴ unabhängig voneinander Halogen, CN, NO₂, -N(R')₂, -CH₂N(R')₂, -OR', -CH₂OR', -SR', -CH₂SR', -COOR, -NRCOR', -CON(R')₂, -OCON(R')₂, COR', -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist, bedeutet,
- x 0 bis 4,
- jeder R³ unabhängig voneinander Halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂ oder eine wahlweise substituierte Gruppe, die aus C₁- bis C₆-aliphatisch, Aryl, Heteroaryl, cycloaliphatisch, heterocycloaliphatisch, ArylC₁-bis C₆-Alkyl, HeteroarylC₁- bis C₆-Alkyl, cycloaliphatischC₁- bis C₆-Alkyl oder heterocycloaliphatischC₁- bis C₆-Alkyl ausgewählt ist,
- y 0 bis 5 und
- jeder R⁵ unabhängig voneinander Halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR' , -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂ oder eine wahlweise substituierte Gruppe, die aus C₁- bis C₆-aliphatisch, Aryl, Heteroaryl, cycloaliphatisch, heterocycloaliphatisch, ArylC₁- bis C6-Alkyl, HeteroarylC₁- bis C₆-Alkyl, cycloaliphatischC₁-bis C₆-Alkyl oder heterocycloaliphatischC₁- bis C₆-Alkyl ausgewählt ist, bedeutet, wobei
- jedes Vorkommen von R' unabhängig voneinander Wasserstoff oder eine wahlweise substituierte C₁- bis C₆-aliphatische Gruppe, einen 3- bis 8gliedrigen gesättigten, teilweise ungesättigten oder vollständig ungesättigten monocyclischen Ring mit 0 bis 3 Heteroatomen, die unabhängig voneinander aus Stickstoff, Sauerstoff oder Schwefel ausgewählt sind, oder ein 8- bis 12gliedriges gesättigtes, teilweise ungesättigtes oder vollständig ungesättigtes bicyclisches Ringsystem mit 0 bis 5 Heteroatomen, die unabhängig voneinander aus Stickstoff, Sauerstoff oder Schwefel ausgewählt sind, bedeutet oder
- zwei Vorkommen von R' mit dem/den Atom/en, an welche/s sie gebunden sind, zusammengenommen werden, um einen wahlweise substituierten 3- bis 12gliedrigen gesättigten, teilweise ungesättigten oder vollständig ungesättigten monocyclischen oder bicyclischen Ring mit 0 bis 4 Heteroatomen, die unabhängig voneinander aus Stickstoff, Sauerstoff oder Schwefel ausgewählt sind, zu bilden, und
d) Entfernen der geeigneten Hydroxylschutzgruppe, um eine Verbindung mit der Formel **Ia** zu bilden,
umfasst.

2. Verfahren nach Anspruch 1, wobei die Estergruppe Trimethylacetyloxy ist.

3. Verfahren nach Anspruch 1 oder 2, wobei das Verfahren weiterhin die Stufe der Bildung eines Salzes der Verbindung mit der Formel **Ia** umfasst.

4. Verfahren nach Anspruch 3, wobei die Verbindung mit der Formel **Ia** als Salz einer Sulfonsäure hergestellt wird.

5. Verfahren nach Anspruch 3, wobei die Verbindung mit der Formel **Ia** als Salz der Methylsulfonsäure oder p-Toluolsulfonsäure hergestellt wird.

6. Verfahren nach Anspruch 3, wobei die Verbindung mit der Formel **Ia** als Salz der Methylsulfonsäure hergestellt wird.

7. Verfahren nach Anspruch 3, wobei die Verbindung mit der Formel **Ia** als Salz einer Dicarbonsäure hergestellt wird.

8. Verfahren nach Anspruch 3, wobei die Verbindung mit der Formel **Ia** als Salz der Oxalsäure, Malonsäure, Bernsteinsäure, Maleinsäure oder Fumarsäure hergestellt wird.

9. Verfahren nach Anspruch 3, wobei die Verbindung mit der Formel **Ia** als Salz der Oxalsäure hergestellt wird.

10. Verfahren nach Anspruch 1 zur Herstellung einer Verbindung mit der Formel **V** aus einer Verbindung mit der Formel **Iaa-1:** 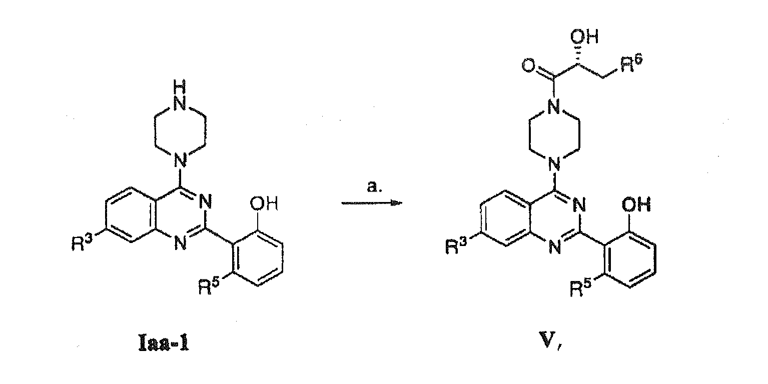 welches die zusätzliche Stufe:
a) Umsetzen der Verbindung mit der Formel **Iaa-1** mit einer geeigneten Säure unter geeigneten Amidkupplungsbedingen,
wobei R⁶ Isopropyl oder tert.-Butyl, R³ Methyl oder Wasserstoff und R⁵ Fluor oder Wasserstoff bedeutet,
umfasst.

11. Verfahren nach Anspruch 10, das ferner die Stufe der Bildung eines Salzes der Verbindung mit der Formel **V** umfasst.

12. Verfahren nach Anspruch 10 oder 11, wobei R⁶ Isopropyl, R³ Methyl und R⁵ Wasserstoff bedeutet.

13. Verfahren nach Anspruch 10 oder 11, wobei R⁶ tert.-Butyl, R³ Methyl und R⁵ Wasserstoff bedeutet.

14. Verfahren nach Anspruch 10 oder 11, wobei R⁶ Isopropyl, R³ Wasserstoff und R⁵ Wasserstoff bedeutet.

15. Verfahren nach Anspruch 10 oder 11, wobei R⁶ tert.-Butyl, R³ Methyl und R⁵ Fluor bedeutet.

## Revendications

1. Procédé de préparation d'un composé de formule Ia : 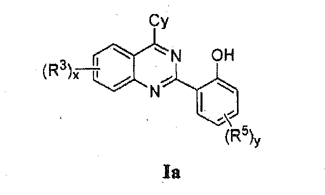 ou d'un sel approprié de celui-ci ; où
Cy est un cycle sélectionné parmi : 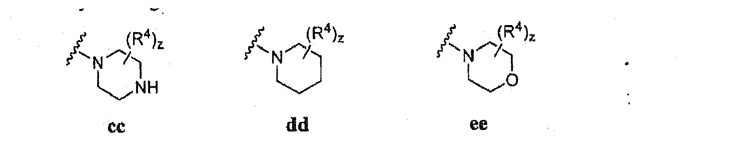 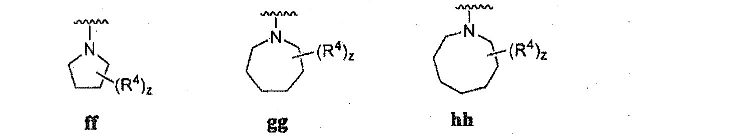 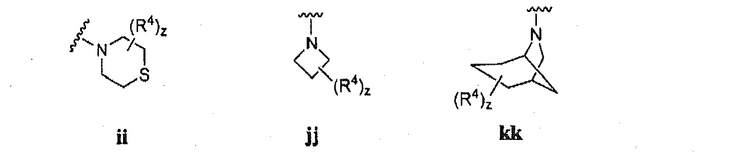 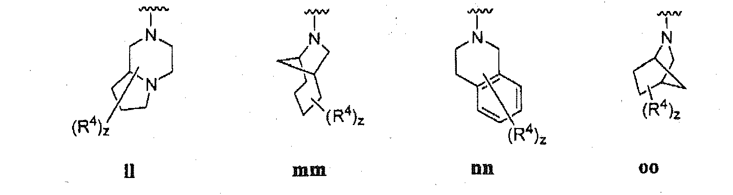 et Cy est facultativement substitué au niveau d'un ou plusieurs atomes de carbone, d'azote ou de soufre substituables par z occurrences indépendantes de -R⁴ ;
chaque z vaut indépendamment 0 à 5 ;
chaque R⁴ est indépendamment halogène, CN, NO₂, - N(R')₂, -CH₂N(R')₂, -OR', -CH₂OR', -SR', - CH₂SR', -COOR' , -NRCOR', -CON(R')₂, -OCON(R')₂, COR' , -NHCOOR', -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
x vaut 0 à 4 ;
chaque R³ est indépendamment halogène, CN, NO₂, - N(R')₂, -CH₂N(R')₂, -OR' , -CH₂OR', -SR', - CH₂SR', -COOR', -NRCOR', -CON(R')₂, -OCON(R')₂, COR' , -NHCOOR' , -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
y vaut 0 à 5 ;
chaque R⁵ est indépendamment halogène, CN, NO₂, - N(R')₂, -CH₂N(R')₂, -OR', -CH₂OR', -SR', - CH₂SR', -NRCOR', -CON (R')₂, -S (O)₂N (R')₂, - OCOR' , -COR' , -CO₂R', -OCON(R')₂, -NR'SO₂R', - OP(O)(OR')₂, -P(O)(OR')₂, -OP(O)₂OR', -P(O)₂OR', -PO(R')₂, -OPO(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ; et
chaque occurrence de R' est indépendamment hydrogène ou un groupe aliphatique en C₁ à C₆ facultativement substitué, un cycle monocyclique de 3 à 8 chaînons saturé, partiellement insaturé ou complètement insaturé ayant de 0 à 3 hétéroatomes sélectionnés indépendamment parmi l'azote, l'oxygène ou le soufre ou un système cyclique bicyclique de 8 à 12 chaînons saturé, partiellement insaturé ou complètement insaturé ayant de 0 à 5 hétéroatomes sélectionnés indépendamment parmi l'azote, l'oxygène ou le soufre ; ou
deux occurrences de R' sont prises conjointement avec le ou les atomes auxquels elles sont liées pour former un cycle monocyclique ou bicyclique de 3 à 12 chaînons saturé, partiellement insaturé ou complètement insaturé ayant de 0 à 4 hétéroatomes sélectionnés indépendamment parmi l'azote, l'oxygène ou le soufre ;
comprenant les étapes consistant à :
(a) fournir un composé de formule **II :** 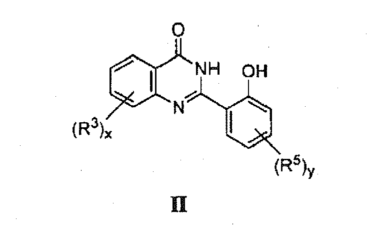 ou un sel approprié de celui-ci ;
où
x vaut 0 à 4 ;
chaque R³ est indépendamment halogène, CN, NO₂, - N(R')₂, -CH₂N(R')₂, -OR' , -CH₂OR' , -SR' , - CH₂SR', -COOR', -NRCOR', -CON(R') 2, -OCON(R')₂, COR' , -NHCOOR' , -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
y vaut 0 à 5 ;
chaque R⁵ est indépendamment halogène, CN, NO₂, - N(R')₂, -CH₂N(R')₂, -OR', -CH₂OR', -SR', - CH₂SR', -NRCOR', -CON(R')₂, -S(O)₂N(R')₂, - OCOR', -COR' , -CO₂R', -OCON(R')₂, -NR'SO₂R', - OP(O)(OR')₂, -P(O)(OR')₂, -OP(O)₂OR', -P(O)₂OR', -PO(R')₂, -OPO(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ; et
chaque occurrence de R' est indépendamment hydrogène ou un groupe aliphatique en C₁ à C₆ facultativement substitué, un cycle monocyclique de 3 à 8 chaînons saturé, partiellement insaturé ou complètement insaturé ayant de 0 à 3 hétéroatomes sélectionnés indépendamment parmi l'azote, l'oxygène ou le soufre ou un système cyclique bicyclique de 8 à 12 chaînons saturé, partiellement insaturé ou complètement insaturé ayant de 0 à 5 hétéroatomes sélectionnés indépendamment parmi l'azote, l'oxygène ou le soufre ; ou
deux occurrences de R' sont prises conjointement avec le ou les atomes auxquels elles sont liées pour former un cycle monocyclique ou bicyclique de 3 à 12 chaînons saturé, partiellement insaturé ou complètement insaturé ayant de 0 à 4 hétéroatomes sélectionnés indépendamment parmi l'azote, l'oxygène ou le soufre ;
et
(b) convertir ledit composé de formule **II** ou un sel approprié de celui-ci en un composé de formule **Ia** ou un sel approprié de celui-ci ;
le procédé comprenant en outre les étapes consistant à :
(a) protéger le groupe hydroxyle du composé **II** avec un groupe protecteur hydroxyle approprié pour former un composé de formule **IIa :** 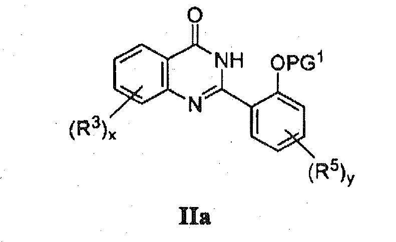 ou un sel approprié de celui-ci ;
où :
OPG¹ est un groupe ester ;
x vaut 0 à 4 ;
chaque R³ est indépendamment halogène, CN, NO₂, - N(R')₂, -CH₂N(R')₂, -OR', -CH₂OR', -SR', - CH₂SR', -COOR', -NRCOR', -CON(R')₂, -OCON(R')₂, COR' , -NHCOOR' , -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
y vaut 0 à 5 ;
chaque R⁵ est indépendamment halogène, CN, NO₂, - N(R')₂, -CH₂N(R')₂, -OR' , -CH₂OR', -SR', - CH₂SR', -NRCOR', -CON(R')₂, -S(O)₂N(R')₂, - OCOR', -COR' , -CO₂R', -OCON(R')₂, -NR'SO₂R' , - OP(O)(OR')₂, -P(O)(OR')₂, -OP(O)₂OR', -P(O)₂OR', -PO(R')₂, -OPO(R')₂ ou un groupe facultativement substitué sélectionné parmi aliphatique en C₁ à C₆, aryle, hétéroaryle, cyclcaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ; et
chaque occurrence de R' est indépendamment hydrogène ou un groupe aliphatique en C₁ à C₆ facultativement substitué, un cycle monocyclique de 3 à 8 chaînons saturé, partiellement insaturé ou complètement insaturé ayant de 0 à 3 hétéroatomes sélectionnés indépendamment parmi l'azote, l'oxygène ou le soufre ou un système cyclique bicyclique de 8 à 12 chaînons saturé, partiellement insaturé ou complètement insaturé ayant de 0 à 5 hétéroatomes sélectionnés indépendamment parmi l'azote, l'oxygène ou le soufre ; ou
deux occurrences de R' sont prises conjointement avec le ou les atomes auxquels elles sont liées pour former un cycle monocyclique ou bicyclique de 3 à 12 chaînons saturé, partiellement insaturé ou complètement insaturé ayant de 0 à 4 hétéroatomes sélectionnés indépendamment parmi l'azote, oxygène ou le soufre ;
(b) convertir la fraction cétone du composé de formule **IIa** ou d'un sel approprié de celui-ci en un groupe partant approprié pour former un composé de formule **IIb :** 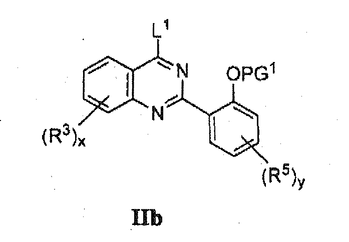 ou un sel approprié de celui-ci ;
où :
OPG¹ est un groupe ester ;
L¹ est un groupe partant approprié ;
x vaut 0 à 4 ;
chaque R³ est indépendamment halogène, CN, NO₂, N(R')₂, -CH₂N(R')₂, -OR' , -CH₂OR', -SR', - CH₂SR', -COOR', -NRCOR' , -CON(R')₂, -OCON(R')₂, COR' , -NHCOOR' , -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
y vaut 0 à 5 ;
chaque R⁵ est indépendamment halogène, CN, NO₂, - N(R')₂, -CH₂N(R')₂, -OR', -CH₂OR', -SR' , - CH₂SR', -NRCOR', -CON(R')₂, -S(O)₂N(R')₂, - OCOR', -COR' , -CO₂R', -OCON(R')₂, -NR'SO₂R', - OP(O)(OR')₂, -P(O) (OR')₂, -OP(O)₂OR', -P(O)₂OR', -PO(R')₂, -OPO(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ; et
chaque occurrence de R' est indépendamment hydrogène ou un groupe aliphatique en C₁ à C₆ facultativement substitué, un cycle monocyclique de 3 à 8 chaînons saturé, partiellement insaturé ou complètement insaturé ayant de 0 à 3 hétéroatomes sélectionnés indépendamment parmi l'azote, l'oxygène ou le soufre ou un système cyclique bicyclique de 8 à 12 chaînons saturé, partiellement insaturé ou complètement insaturé ayant de 0 à 5 hétéroatomes sélectionnés indépendamment parmi l'azote, l'oxygène ou le soufre ; ou
deux occurrences de R' sont prises conjointement avec le ou les atomes auxquels elles sont liées pour former un cycle monocyclique ou bicyclique de 3 à 12 chaînons saturé, partiellement insaturé ou complètement insaturé ayant de 0 à 4 hétéroatomes sélectionnés indépendamment parmi l'azote, l'oxygène ou le soufre ;
(c) déplacer ledit groupe partant approprié avec une fraction Cy appropriée pour former un composé de formule **IIc :** 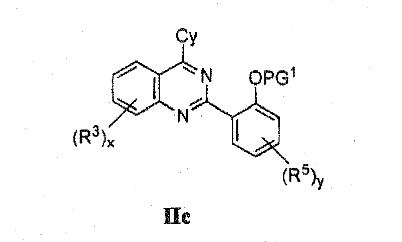 ou un sel approprié de celui-ci ;
où :
OPG¹ est un groupe ester ;
Cy est un cycle sélectionné parmi : 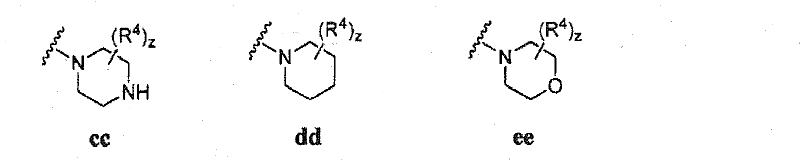 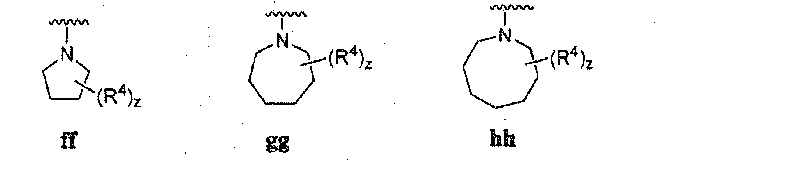 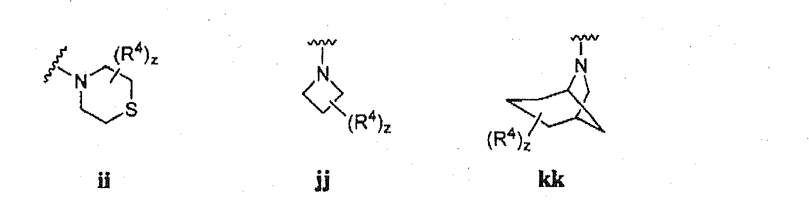 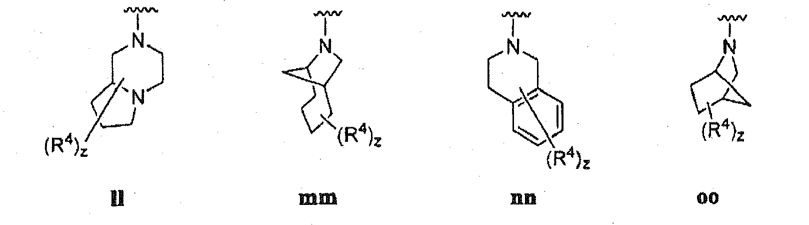 et Cy est facultativement substitué au niveau d'un ou plusieurs atomes de carbone, d'azote ou de soufre substituables par z occurrences indépendantes de -R⁴ ;
chaque z vaut indépendamment 0 à 5 ;
chaque R⁴ est indépendamment halogène, CN, NO₂, - N(R')₂, -CH₂N(R')₂, -OR', -CH₂OR', -SR', - CH₂SR', -COOR', -NRCOR', -CON(R')₂, -OCON(R')₂, COR' , -NHCOOR' , -SO₂R', -SO₂N(R') 2 ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
x vaut 0 à 4 ;
chaque R³ est indépendamment halogène, CN, NO₂, - N(R')₂, -CH₂N(R')₂, -OR', -CH₂OR', -SR', - CH₂SR', -COOR', -NRCOR' , -CON(R')₂, -OCON(R')₂, COR', -NHCOOR', -SO₂R', -SO₂N(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ;
y vaut 0 à 5 ;
chaque R⁵ est indépendamment halogène, CN, NO₂, - N(R')₂, -CH₂N(R')₂, -OR', -CH₂OR', -SR', - CH₂SR', -NRCOR', -CON(R')₂, -S(O)₂N(R')₂, - OCOR', -COR', -CO₂R', -OCON(R')₂, -NR'SO₂R', - OP(O)(OR')₂, -P (O) (OR')₂, -OP(O)₂OR', -P(O)₂OR', -PO(R')₂, -OPO(R')₂ ou un groupe facultativement substitué sélectionné parmi aliphatique en C₁ à C₆, aryle, hétéroaryle, cycloaliphatique, hétérocycloaliphatique, aryle alkyle en C₁ à C₆, hétéroaryle alkyle en C₁ à C₆, cycloaliphatique alkyle en C₁ à C₆ ou hétérocycloapliphatique alkyle en C₁ à C₆ ; et
chaque occurrence de R' est indépendamment hydrogène ou un groupe aliphatique en C₁ à C₆ facultativement substitué, un cycle monocyclique de 3 à 8 chaînons saturé, partiellement insaturé ou complètement insaturé ayant de 0 à 3 hétéroatomes sélectionnés indépendamment parmi l'azote, l'oxygène ou le soufre ou un système cyclique bicyclique de 8 à 12 chaînons saturé, partiellement insaturé ou complètement insaturé ayant de 0 à 5 hétéroatomes sélectionnés indépendamment parmi l'azote, l'oxygène ou le soufre ; ou
deux occurrences de R' sont prises conjointement avec le ou les atomes auxquels elles sont liées pour former un cycle monocyclique ou bicyclique de 3 à 12 chaînons saturé, partiellement insaturé ou complètement insaturé ayant de 0 à 4 hétéroatomes sélectionnés indépendamment parmi l'azote, l'oxygène ou le soufre ;
et
(d) supprimer le groupe protecteur hydroxyle approprié pour former un composé de formule **Ia.**

2. Procédé selon la revendication 1, dans lequel ledit groupe ester est triméthylacétyloxy.

3. Procédé selon la revendication 1 ou 2, dans lequel ledit procédé comprend en outre l'étape de formation d'un sel du composé de formule **Ia.**

4. Procédé selon la revendication 3, dans lequel le composé de formule **Ia** est produit en tant que sel d'un acide sulfonique.

5. Procédé selon la revendication 3, dans lequel le composé de formule **Ia** est produit en tant que sel d'acide méthylsulfonique ou d'acide p-toluènesulfonique.

6. Procédé selon la revendication 3, dans lequel le composé de formule **Ia** est produit en tant que sel d'acide méthylsulfonique.

7. Procédé selon la revendication 3, dans lequel le composé de formule **Ia** est produit en tant que sel d'un acide dicarboxylique.

8. Procédé selon la revendication 3, dans lequel le composé de formule **Ia** est produit en tant que sel d'acide oxalique, d'acide malonique, d'acide succinique, d'acide maléique ou d'acide fumarique.

9. Procédé selon la revendication 3, dans lequel le composé de formule **Ia** est produit en tant que sel d'acide oxalique.

10. Procédé selon la revendication 1 pour préparer un composé de formule **V** à partir d'un composé de formule **Iaa-1 :** 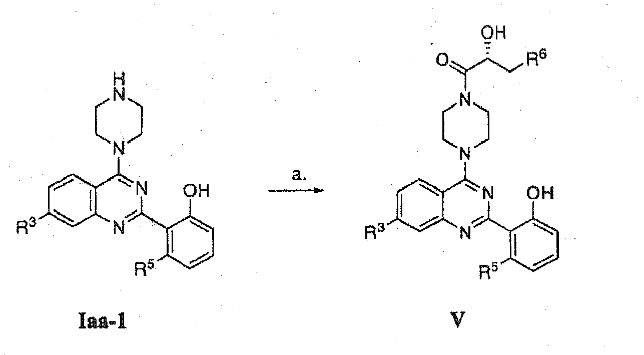 comprenant l'étape supplémentaire consistant à :
(a) faire réagir le composé de formule **Iaa-1** avec un acide approprié dans des conditions de couplage d'amides appropriées ;
où R⁶ est isopropyle ou t-butyle, R³ est méthyle ou hydrogène et R⁵ est fluor ou hydrogène.

11. Procédé selon la revendication 10, dans lequel ledit procédé comprend en outre l'étape de formation d'un sel du composé de formule **V.**

12. Procédé selon la revendication 10 ou 11, dans lequel R⁶ est isopropyle, R³ est méthyle et R⁵ est hydrogène.

13. Procédé selon la revendication 10 ou 11, dans lequel R⁶ est t-butyle, R³ est méthyle et R⁵ est hydrogène.

14. Procédé selon la revendication 10 ou 11, dans lequel R⁶ est isopropyle, R³ est hydrogène et R⁵ est hydrogène.

15. Procédé selon la revendication 10 ou 11, dans lequel R⁶ est t-butyle, R³ est méthyle et R⁵ est fluor.
